(19) Europäisches Patentamt
European Patent Office
Office européen des brevets

(11) Veröffentlichungsnummer: **0 567 968 A1**

# (12) EUROPÄISCHE PATENTANMELDUNG

(21) Anmeldenummer: **93106726.8**

(22) Anmeldetag: **26.04.93**

(51) Int. Cl.[5]: **C07D 207/26**, C07D 207/38, A61K 31/40

(30) Priorität: **28.04.92 DE 4213931**

(43) Veröffentlichungstag der Anmeldung:
**03.11.93 Patentblatt 93/44**

(84) Benannte Vertragsstaaten:
**AT BE CH DE DK ES FR GB GR IE IT LI LU NL PT SE**

(71) Anmelder: **Dr. Karl Thomae GmbH**

**D-88397 Biberach(DE)**

(72) Erfinder: **linz, Günter, Dr.**
**Erlenweg 8**
**W-7951 Mittelbiberach(DE)**
Erfinder: **Austel, Volkhard, Dr.**
**Kapellenweg 7**
**W-7950 Biberach 1(DE)**
Erfinder: **Himmelsbach, Frank, dr.**
**Ahornweg 16**
**W-7951 Mittelbiberach(DE)**
Erfinder: **Weisenberger, Johannes, Dr.**
**Haydnweg 5**
**W-7950 Biberach 1(DE)**
Erfinder: **Müller, Thomas, Dr.**
**Alter Postplatz 17**
**W-7950 Biberach 1(DE)**
Erfinder: **Pieper, Helmut, Dr.**
**Kapellenweg 5**
**W-7950 Biberach 1(DE)**
Erfinder: **Seewaldt-Becker, Elke, Dr.**
**Stresemannstrasse 40**
**W-88400 Biberach(DE)**

## (54) Cyclische Iminoderivate, diese Verbindungen enthaltende Arzneimittel und Verfahren zu ihrer Herstellung.

(57) Die Erfindung betrifft cyclische Iminoderivate der allgemeinen Formel

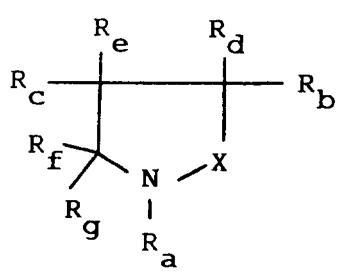

,(I)

in der
X und $R_a$ bis $R_g$ wie im Anspruch 1 definiert sind, deren Tautomere, deren Stereoisomere einschließlich deren Gemische und deren Salze, insbesondere deren physiologisch vertragliche Salze mit anorganischen oder organischen Säuren oder Basen, welche wertvolle pharmakologische Eigenschaften aufweisen, vorzugsweise aggregationshemmende Wirkungen, die Verbindungen enthaltende Arzneimittel und Verfahren zu ihrer Herstellung.

EP 0 567 968 A1

Die Erfindung betrifft cyclische Iminoderivate der allgemeinen Formel

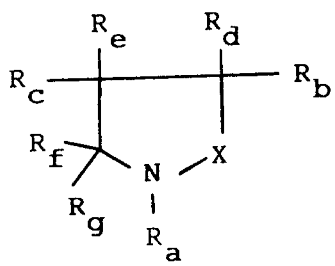

, (I)

deren Stereoisomere, deren Gemische und deren Salze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, diese Verbindungen enthaltende Arzneimittel und Verfahren zu ihrer Herstellung.

In der obigen allgemeinen Formel I bedeutet

einer der Reste $R_a$ oder $R_b$ eine A-B-Gruppe und

der andere der Reste $R_a$ oder $R_b$ eine F-E-D-Gruppe, in denen

A eine Aminogruppe, eine Aminoalkylgruppe mit 1 bis 4 Kohlenstoffatomen im Alkylteil, eine Amidino- oder Guanidinogruppe, wobei in den vorstehend erwähnten Amino-, Aminoalkyl-, Amidino- oder Guanidinogruppen eine Amino- oder Iminogruppe durch eine Alkylgruppe mit 1 bis 4 Kohlenstoffatomen oder durch eine in vivo abspaltbare Gruppe substituiert und eine Aminogruppe zusätzlich durch eine weitere Alkylgruppe mit 1 bis 4 Kohlenstoffatomen oder durch eine Aralkylgruppe substituiert sein kann,

B eine Phenylengruppe, die durch ein Fluor-, Chlor- oder Bromatom, durch eine oder zwei Alkylgruppen, durch eine Trifluormethyl-, Hydroxy-, Alkoxy-, Alkylsulfenyl-, Alkylsulfinyl-, Alkylsulfonyl-, Nitro-, $(R_1)_2N$-, N-Alkylcarbonyl-$NR_1$- oder N-Alkansulfonyl-$NR_1$-Gruppe, wobei $R_1$ ein Wasserstoffatom, eine Alkyl- oder Aralkylgruppe darstellt, substituiert sein kann,

eine Pyridinylen-, Pyrimidinylen-, Pyrazinylen-, Pyridazinylen- oder Triazinylengruppe, die jeweils im Kohlenstoffgerüst durch eine Alkylgruppe substituiert und in denen zusätzlich eine oder zwei -CH=N-Gruppen jeweils durch eine -CO-$NR_1$-Gruppe ersetzt sein können, wobei $R_1$ wie vorstehend erwähnt definiert ist und zwischen dem Stickstoffatom der -CO-$NR_1$-Gruppe und dem Stickstoffatom des Restes A oder des cyclischen Iminorestes, an den der Rest B geknüpft ist, mindestens 2 Kohlenstoffatome liegen müssen,

eine 1,3-Pyrrolidinylen-, 1,3-Piperidinylen-, 1,4-Piperidinylen-, 1,3-Hexahydroazepinylen-, 1,4-Hexahydroazepinylen-, 1,4-Piperazinylen- oder 1,4-Homopiperazinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf, und

eine Cycloalkylengruppe mit 5 bis 7 Kohlenstoffatomen, in der eine Methylengruppe durch eine -$NR_1$-Gruppe, in der $R_1$ wie vorstehend erwähnt definiert ist, ersetzt sein kann, wobei zusätzlich zwischen dem Stickstoffatom der -$NR_1$-Gruppe und einem Stickstoffatom des Restes A oder des cyclischen Iminorestes mindestens zwei Kohlenstoffatome liegen müssen, und

eine Indanyliden-, 1,2,3,4-Tetrahydronaphthyliden- oder 5H-Benzocycloheptenylidengruppe oder

A und B zusammen eine gegebenenfalls in 1-Stellung durch den Rest $R_1$ oder durch eine in vivo abspaltbare Gruppe substituierte Pyrrolidinyl-, Piperidinyl-, Hexahydroazepinyl- oder Piperazinogruppe, wobei $R_1$ wie vorstehend erwähnt definiert ist,

D eine Phenylengruppe, die durch ein Fluor-, Chlor- oder Bromatom, durch eine oder zwei Alkylgruppen, durch eine Trifluormethyl-, Hydroxy-, Alkoxy-, Alkylsulfenyl-, Alkylsulfinyl-, Alkylsulfonyl-, Nitro-, $(R_1)_2N$-, N-Alkylcarbonyl-$NR_1$-, N-Alkansulfonyl-$NR_1$-, Carboxymethoxy- oder Alkoxycarbonylmethoxygruppe substituiert sein kann, wobei $R_1$ wie vorstehend erwähnt definiert ist,

eine Cycloalkylengruppe mit 5 bis 7 Kohlenstoffatomen,

eine Pyridinylen-, Pyrimidinylen-, Pyrazinylen-, Pyridazinylen- oder Triazinylengruppe, die jeweils im Kohlenstoffgerüst durch eine Alkylgruppe substituiert sein kann, wobei zusätzlich eine -CH=N-Gruppe durch eine -CO-$NR_2$-Gruppe ersetzt sein kann, wobei $R_2$ ein Wasserstoffatom, eine Alkyl- oder Aralkylgruppe oder auch eine Bindung mit dem Rest E darstellt, wenn sich immer zwischen einem Heteroatom des Restes E oder dem Stickstoffatom des cyclischen Iminorestes mit dem der Rest D verknüpft ist und dem

Stickstoffatom der $-CONR_2$-Gruppe mindestens zwei Kohlenstoffatome befinden,

eine 1,3-Pyrrolidinylen-, 1,3-Piperidinylen-, 1,4-Piperidinylen-, 1,3-Hexahydroazepinylen- oder 1,4-Hexahydroazepinylen-, 1,4-Piperazinylen- oder 1,4-Homopiperazinylengruppe, welche über die Position 1 mit dem Rest E oder mit dem cyclischen Iminorest verknüpft sein können und in denen eine zu dem Stickstoffatom benachbarte Methylengruppe durch eine Carbonylgruppe ersetzt sein kann, wobei jedoch die Verknüpfung nicht über eine Stickstoff-Stickstoff-Bindung erfolgen darf,

eine Cycloalkylengruppe mit 5 bis 7 Kohlenstoffatomen, in der eine Methylengruppe durch eine $-NR_1$-Gruppe ersetzt ist, wobei $R_1$ wie vorstehend erwähnt definiert ist,

E eine gegebenenfalls durch eine Hydroxy-, Alkoxy-, $(R_1)_2N$-, Alkyl-CO-$NR_1$-, Aralkyl-CO-$NR_1$-, Aryl-CO-$NR_1$-, $C_{1-5}$ -Alkyl-$SO_2$-$NR_1$-, Arylalkyl-$SO_2$-$NR_1$-, Aryl-$SO_2$-$NR_1$-, $(R_1)_2N$-CO-$NR_1$-, Alkoxy-CO-$NR_1$- oder Aralkoxy-CO-$NR_1$-Gruppe substituierte geradkettige oder verzweigte Alkylengruppe mit 1 bis 5 Kohlenstoffatomen, in denen die Reste $R_1$, die gleich oder verschieden sein können, wie eingangs erwähnt definiert sind, eine geradkettige oder verzweigte Alkenylengruppe mit 2 bis 5 Kohlenstoffatomen, wobei in einer Alkylengruppe mit 2 bis 4 Kohlenstoffatomen die mit dem Rest D verknüpfte Methylengruppe durch ein Sauerstoff- oder Schwefelatom, durch eine Sulfinyl-, Sulfonyl-, Imino-, Alkylimino-, Aralkylimino-, Alkylcarbonylimino-, Aralkylcarbonylimino-, Arylcarbonylimino-, Alkansulfonylimino-, Arylalkansulfonylimino-, Arylsulfonylimino-, Carboxymethylimino-, Alkoxycarbonylmethylimino- oder Aralkoxycarbonylmethyliminogruppe ersetzt sein kann, und

F eine in vivo zu einer Carboxylgruppe metabolisierbare Gruppe, eine Carboxylgruppe, eine Sulfo-, Phosphono-, O-Alkylphosphono- oder Tetrazol-5-ylgruppe darstellen, und entweder

(a) X eine Methylen- oder Carbonylgruppe,

$R_d$ und $R_e$ zusammen eine weitere Kohlenstoff-Kohlenstoff-Bindung,

$R_c$ ein Wasserstoffatom, eine Alkylgruppe mit 1 bis 5 Kohlenstoffatomen, eine Arylgruppe, eine Hydroxygruppe, eine geradkettige oder verzweigte Alkoxygruppe mit 1 bis 5 Kohlenstoffatomen, wobei eine Alkoxygruppe mit 1 bis 3 Kohlenstoffatomen in 1-, 2- oder 3-Stellung durch eine Carboxy-, Alkoxycarbonyl-, Aminocarbonyl-, Alkylaminocarbonyl-, Dialkylaminocarbonyl-, Cycloalkylaminocarbonyl-, Cycloalkylalkylaminocarbonyl-, Piperidinocarbonyl-, Morpholinocarbonyl-, Thiomorpholinocarbonyl-, 1-Oxidothiomorpholinocarbonyl- oder 1,1-Dioxidothiomorpholinocarbonylgruppe substituiert sein kann, eine Aralkoxy-, Alkylamino-, Dialkylamino-, Aralkylamino-, N-Alkyl-aralkylamino-, Arylamino-, N-Alkyl-arylamino-, Pyrrolidino-, Piperidino- oder Hexamethyleniminogruppe, wobei die Methylengruppe in 4-Stellung einer Piperidinogruppe durch ein Sauerstoff- oder Schwefelatom, durch eine Sulfinyl-, Sulfonyl-, Imino-, Alkylimino-, Aralkylimino-, Alkylcarbonylimino-, Aralkylcarbonylimino-, Arylcarbonylimino-, Alkansulfonylimino-, Arylalkansulfonylimino- oder Arylsulfonyliminogruppe ersetzt sein kann,

$R_f$ ein Wasserstoffatom, eine geradkettige oder verzweigte Alkylgruppe mit 1 bis 5 Kohlenstoffatomen, eine Aralkyl- oder Arylgruppe und

$R_g$ ein Wasserstoffatom oder eine Alkylgruppe oder

(b) X eine Carbonylgruppe,

$R_c$ und $R_e$ zusammen mit dem dazwischen liegenden Kohlenstoffatom eine Carbonylgruppe,

$R_d$ eine gegebenenfalls durch eine Aryl-, Carboxy-, Alkoxycarbonyl-, Aminocarbonyl-, Alkylaminocarbonyl-, Dialkylaminocarbonyl-, Cycloalkylaminocarbonyl-, Cycloalkylalkylaminocarbonyl-, Piperidinocarbonyl-, Morpholinocarbonyl-, Thiomorpholinocarbonyl-, 1-Oxidothiomorpholinocarbonyl- oder 1,1-Dioxidothiomorpholinocarbonylgruppe substituierte Alkylgruppe,

$R_f$ ein Wasserstoffatom, eine Alkyl- oder Arylgruppe und

$R_g$ ein Wasserstoffatom oder eine Alkylgruppe oder

(c) X eine Methylengruppe,

$R_f$ und $R_g$ zusammen mit dem dazwischen liegenden Kohlenstoffatom eine Carbonylgruppe,

$R_c$ ein Wasserstoffatom oder auch, wenn B oder D eine 1,3-Pyrrolidinylen-, 1,3-Piperidinylen-, 1,4-Piperidinylen-, 1,3-Hexahydroazepinylen-, 1,4-Hexahydroazepinylen-, 1,4-Piperazinylen- oder 1,4-Homopiperazinylengruppe, welche über die Position 1 mit dem cyclischen Iminorest verknüpft sind, darstellt, eine Arylgruppe und

$R_d$ und $R_e$ jeweils ein Wasserstoffatom oder zusammen eine weitere Kohlenstoff-Kohlenstoff-Bindung oder

(d) X eine Methylen- oder Carbonylgruppe und

$R_c$ bis $R_f$ jeweils ein Wasserstoffatom,

wobei unter "eine in vivo zu einer Carboxylgruppe metabolisierbare Gruppe" und unter "eine in vivo abspaltbare Gruppe" beispielsweise eine Estergruppe der Formeln

- CO - OR',

- CO - O - (HCR'') - O - CO - R''' und
- CO - O - (HCR'') - O - CO - OR''',

in denen
R' eine geradkettige oder verzweigte Alkylgruppe mit 1 bis 6 Kohlenstoffatomen, wobei die Methylgruppe durch eine $(R_1)_2$NCO-Gruppe, in der $R_1$ wie eingangs definiert ist, durch eine Pyridyl-, Cycloalkylaminocarbonyl-, Cycloalkylalkylaminocarbonyl-, Pyrrolidinocarbonyl-, Piperidinocarbonyl-, Morpholinocarbonyl-, Thiomorpholinocarbonyl-, 1-Oxidothiomorpholinocarbonyl- oder 1,1-Dioxidothiomorpholinocarbonylgruppe, eine $C_{1-3}$-Alkylgruppe in 1-, 2- oder 3-Stellung durch eine Arylgruppe oder eine $C_{2-3}$-Alkylgruppe in 2- oder 3-Stellung durch eine Pyrrolidin-2-on-1-yl-, Morpholino-, Thiomorpholino-, 1-Oxido-thiomorpholino- oder 1,1-Dioxidothiomorpholinogruppesubstituiert sein kann, eine Alkenylgruppe mit 1 bis 4 Kohlenstoffatomen, eine Cycloalkyl-, Cycloalkenyl-, Cycloalkylalkyl-, Methoxymethyl- oder Cinnamylgruppe,
R'' ein Wasserstoffatom oder eine Alkylgruppe und
R''' eine geradkettige oder verzweigte Alkylgruppe mit 1 bis 6 Kohlenstoffatomen, eine Cycloalkyl-, Cycloalkylalkyl- oder Arylalkylgruppe bedeuten,
wobei eine nach der Abspaltung so gebildete Carboxylgruppe, falls diese an ein Stickstoffatom des Restes A gebunden ist, unter Freisetzung der Amino- oder Iminogruppe decarboxyliert werden kann,
sowie, soweit nichts anderes erwähnt wurde,
unter dem vorstehend erwähnten Begriff "eine Alkylgruppe" eine geradkettige oder verzweigte Alkylgruppe mit 1 bis 3 Kohlenstoffatomen,
unter "eine Cycloalkylgruppe" eine Cycloalkylgruppe mit 5 bis 7 Kohlenstoffatomen,
unter "eine Cycloalkenylgruppe" eine Cycloalkenylgruppe mit 5 bis 7 Kohlenstoffatomen,
unter "eine Alkoxygruppe" eine geradkettige oder verzweigte Alkoxygruppe mit 1 bis 3 Kohlenstoffatomen und
unter "eine Arylgruppe" eine durch Fluor-, Chlor- oder Bromatome, durch Alkylgruppen mit 1 bis 5 Kohlenstoffatomen, durch Hydroxy-, Alkoxy-, Phenylalkoxy-, Trifluormethyl-, Mercapto-, Alkylsulfenyl-, Alkylsulfinyl-, Alkylsulfonyl-, Amino-, Alkylamino-, Dialkylamino-, Alkylcarbonylamino-, Phenylalkylcarbonylamino-, Phenylcarbonylamino-, Alkoxycarbonylamino-, Alkylsulfonylamino-, Phenylsulfonylamino-, N-Alkylcarbonyl-alkylamino-, N-Phenylalkylcarbonyl-alkylamino-, N-Phenylcarbonyl-alkylamino-, N-Alkoxycarbonyl-alkylamino-, N-Alkylsulfonyl-alkylamino-, N-Phenylsulfonylalkylamino-, Cyano-, Aminocarbonyl-, Alkylaminocarbonyl-, Dialkylaminocarbonyl-, Aminosulfonyl-, Alkylaminosulfonyl-, Dialkylaminosulfonyl-, Alkylcarbonyl-, Phenylalkylcarbonyl-, Phenylcarbonyl-, Carboxy-, Sulfo-, Alkoxycarbonyl-, Aminocarbonylamino-, N-Aminocarbonylalkylamino- oder Aminoalkylgruppen mono-, di- oder trisubstituierte Phenylgruppe sein kann, wobei die Substituenten gleich oder verschieden sein können und die Aminogruppe bei den vorstehend erwähnten Aminocarbonylamino-, N-Aminocarbonyl-alkylamino- oder Aminoalkylgruppen zusätzlich durch Alkyl- oder Phenylalkylgruppen mono- oder disubstituiert sein kann,
zu verstehen ist.

Bevorzugte Verbindungen der obigen allgemeinen Formel I sind jedoch diejenigen, in denen
einer der Reste $R_a$ oder $R_b$ eine A-B-Gruppe und
der andere der Reste $R_a$ oder $R_b$ eine F-E-D-Gruppe, in denen

A eine Aminoalkylgruppe mit 1 bis 4 Kohlenstoffatomen im Alkylteil, eine Amino- oder Amidinogruppe, wobei in den vorstehend erwähnten Amino-, Aminoalkyl- oder Amidinogruppen eine Amino- oder Iminogruppe durch eine Alkylgruppe oder durch eine in vivo abspaltbare Gruppe substituiert sein kann,

B eine Phenylengruppe, die durch ein Fluor-, Chlor- oder Bromatom, durch eine Alkylgruppe, durch eine Trifluormethyl-, Hydroxy-, Alkoxy-, Alkylsulfenyl-, Alkylsulfinyl-, Alkylsulfonyl-, $(R_1)_2$N-, N-Alkylcarbonyl-$NR_1$- oder N-Alkansulfonyl-$NR_1$-Gruppe, wobei $R_1$ ein Wasserstoffatom, eine Alkyl- oder Phenylalkylgruppe darstellt, substituiert sein kann,

eine Pyridinylen-, Pyrimidinylen-, Pyrazinylen- oder Pyridazinylengruppe, die jeweils im Kohlenstoffgerüst durch eine Alkylgruppe substituiert sein können,

eine 1,3-Pyrrolidinylen-, 1,3-Piperidinylen-, 1,4-Piperidinylen-, 1,4-Piperazinylen- oder 1,4-Homopiperazinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf, und

eine Cycloalkylengruppe, in der eine Methylengruppe durch eine $-NR_1$-Gruppe, in der $R_1$ wie vorstehend erwähnt definiert ist, ersetzt sein kann, wobei zusätzlich zwischen dem Stickstoffatom der $-NR_1$-Gruppe und einem Stickstoffatom des Restes A oder des cyclischen Iminorestes mindestens zwei Kohlenstoffatome liegen müssen, und

eine Indanyliden- oder 1,2,3,4-Tetrahydronaphthylidengruppe, wobei jeweils der aromatische Kern mit dem cyclischen Iminorest verknüpft ist, oder

A und B zusammen eine gegebenenfalls in 1-Stellung durch den Rest $R_1$ oder durch eine in vivo abspaltbare Gruppe substituierte Pyrrolidinyl-, Piperidinyl- oder Piperazinogruppe, wobei $R_1$ wie vorstehend erwähnt definiert ist,

D eine Phenylengruppe, die durch ein Fluor-, Chlor- oder Bromatom, durch eine Alkylgruppe, durch eine Trifluormethyl-, Hydroxy-, Alkoxy-, Alkylsulfenyl-, Alkylsulfinyl-, Alkylsulfonyl-, $(R_1)_2N$-, N-Alkylcarbonyl-$NR_1$-, N-Alkansulfonyl-$NR_1$-, Carboxymethoxy- oder Alkoxycarbonylmethoxygruppe substituiert sein kann, wobei $R_1$ wie vorstehend erwähnt definiert ist, substituiert sein kann,

eine Cycloalkylengruppe,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine -CH=N-Gruppe durch eine -CO-$NR_2$-Gruppe ersetzt sein kann, wobei $R_2$ ein Wasserstoffatom, eine Alkyl- oder Phenylalkylgruppe oder auch eine Bindung mit dem Rest E darstellt, wenn sich immer zwischen einem Heteroatom des Restes E oder dem Stickstoffatom des cyclischen Iminorestes mit dem der Rest D verknüpft ist und dem Stickstoffatom der -$CONR_2$-Gruppe mindestens zwei Kohlenstoffatome befinden,

eine 1,3-Pyrrolidinylen-, 1,3-Piperidinylen-, 1,4-Piperidinylen-, 1,4-Piperazinylen- oder 1,4-Homopiperazinylengruppe, welche über die Position 1 mit dem Rest E oder mit dem cyclischen Iminorest verknüpft sein können und in denen eine zu dem Stickstoffatom benachbarte Methylengruppe durch eine Carbonylgruppe ersetzt sein kann, wobei jedoch die Verknüpfung nicht über eine Stickstoff-Stickstoff-Bindung erfolgen darf,

eine Cycloalkylengruppe, in der eine Methylengruppe durch eine -$NR_1$-Gruppe ersetzt ist, wobei $R_1$ wie vorstehend erwähnt definiert ist,

E eine gegebenenfalls durch eine Hydroxy-, Alkoxy-, Amino-, Alkylamino-, Dialkylamino-, Alkyl-CO-$NR_1$-, Aryl-CO-$NR_1$-, $C_{1-5}$-Alkyl-$SO_2$-$NR_1$- oder Aryl-$SO_2$-$NR_1$-Gruppe substituierte geradkettige oder verzweigte Alkylengruppe mit 1 bis 5 Kohlenstoffatomen, in denen $R_1$ wie vorstehend erwähnt definiert ist, eine geradkettige oder verzweigte Alkenylengruppe mit 2 bis 5 Kohlenstoffatomen, wobei in einer Alkylengruppe mit 2 bis 4 Kohlenstoffatomen die mit dem Rest D verknüpfte Methylengruppe durch ein Sauerstoff- oder Schwefelatom, durch eine Sulfinyl-, Sulfonyl-, Imino-, Alkylimino-, Phenylalkylimino-, Alkylcarbonylimino-, Carboxymethylimino- oder Alkoxycarbonylmethyliminogruppe ersetzt sein kann, und

F eine in vivo zu einer Carboxylgruppe metabolisierbare Gruppe, eine Carboxylgruppe, eine Sulfo-, Phosphono-, O-Alkylphosphono- oder Tetrazol-5-ylgruppe darstellen, und entweder

(a) X eine Methylen- oder Carbonylgruppe,

$R_d$ und $R_e$ zusammen eine weitere Kohlenstoff-Kohlenstoff-Bindung,

$R_c$ ein Wasserstoffatom, eine Alkylgruppe mit 1 bis 5 Kohlenstoffatomen, eine Phenylgruppe, eine Hydroxygruppe, eine geradkettige oder verzweigte Alkoxygruppe mit 1 bis 5 Kohlenstoffatomen, wobei eine Methoxygruppe durch eine Carboxy-, Alkoxycarbonyl-, Aminocarbonyl-, Alkylaminocarbonyl-, Dialkylaminocarbonyl-, Piperidinocarbonyl-, Morpholinocarbonyl-, Thiomorpholinocarbonyl-, 1-Oxidothiomorpholinocarbonyl- oder 1,1-Dioxidothiomorpholinocarbonylgruppe substituiert sein kann, eine Phenylalkoxy-, Alkylamino-, Dialkylamino-, Phenylalkylamino-, Pyrrolidino- oder Piperidinogruppe, wobei die Methylengruppe in 4-Stellung einer Piperidinogruppe durch ein Sauerstoff- oder Schwefelatom, durch eine Sulfinyl-, Sulfonyl-, Imino-, Alkylimino- oder Alkylcarbonyliminogruppe ersetzt sein kann,

$R_f$ ein Wasserstoffatom, eine geradkettige oder verzweigte Alkylgruppe oder eine Phenylgruppe und

$R_g$ ein Wasserstoffatom oder eine Alkylgruppe oder

(b) X eine Carbonylgruppe,

$R_c$ und $R_e$ zusammen mit dem dazwischen liegenden Kohlenstoffatom eine Carbonylgruppe,

$R_d$ eine Alkylgruppe, eine Phenylalkylgruppe oder eine gegebenenfalls durch eine Carboxy-, Alkoxycarbonyl-, Aminocarbonyl-, Alkylaminocarbonyl-, Dialkylaminocarbonyl-, Piperidinocarbonyl-, Morpholinocarbonyl-, Thiomorpholinocarbonyl-, 1-Oxidothiomorpholinocarbonyl- oder 1,1-Dioxidothiomorpholinocarbonylgruppe substituierte Methylgruppe,

$R_f$ ein Wasserstoffatom, eine Alkyl- oder Phenylgruppe und

$R_g$ ein Wasserstoffatom oder eine Methylgruppe oder

(c) X eine Methylengruppe,

$R_f$ und $R_g$ zusammen mit dem dazwischen liegenden Kohlenstoffatom eine Carbonylgruppe,

$R_c$ ein Wasserstoffatom oder auch, wenn B oder D eine 1,3-Pyrrolidinylen-, 1,3-Piperidinylen-, 1,4-Piperidinylen-, 1,4-Piperazinylen- oder 1,4-Homopiperazinylengruppe, welche über die Position 1 mit dem cyclischen Iminorest verknüpft sind, wobei jedoch die Verbindung nicht über eine Stickstoff-Stickstoff-Bindung erfolgen darf, darstellt, eine Phenylgruppe und

$R_d$ und $R_e$ jeweils ein Wasserstoffatom oder zusammen eine weitere Kohlenstoff-Kohlenstoff-Bindung

oder
(d) X eine Methylen- oder Carbonylgruppe und
$R_c$ bis $R_f$ jeweils ein Wasserstoffatom bedeuten,
wobei unter "eine in vivo zu einer Carboxylgruppe metabolisierbare Gruppe" und unter "eine in vivo abspaltbare Gruppe" beispielsweise eine Estergruppe der Formeln

- CO - OR',
- CO - O - (HCR'') - O - CO - R''' und
- CO - O - (HCR'') - O - CO - OR''',

in denen
R' eine geradkettige oder verzweigte Alkylgruppe mit 1 bis 6 Kohlenstoffatomen, wobei die Methylgruppe durch eine Pyridyl-, Aminocarbonyl-, Alkylaminocarbonyl-, Dialkylaminocarbonyl-, Morpholinocarbonyl-, Thiomorpholinocarbonyl-, 1-Oxidothiomorpholinocarbonyl- oder 1,1-Dioxidothiomorpholinocarbonylgruppe, eine $C_{1-3}$-Alkylgruppe in 1-, 2- oder 3-Stellung durch eine Phenylgruppe oder eine $C_{2-3}$-Alkylgruppe in 2- oder 3-Stellung durch eine Morpholino-, Thiomorpholino-, 1-Oxido-thiomorpholino- oder 1,1-Dioxidothiomorpholinogruppe substituiert sein kann, eine Allyl-, Cycloalkyl- oder Cinnamylgruppe,
R'' ein Wasserstoffatom oder eine Alkylgruppe und
R''' eine geradkettige oder verzweigte Alkylgruppe mit 1 bis 6 Kohlenstoffatomen, eine Cycloalkyl-, Cycloalkylalkyl- oder Phenylalkylgruppe bedeuten, zu verstehen ist, wobei eine nach der Abspaltung so gebildete Carboxylgruppe, falls diese an ein Stickstoffatom des Restes A gebunden ist, unter Freisetzung der Amino- oder Iminogruppe decarboxyliert werden kann,
wobei die bei der Definiton der vorstehenden Reste erwähnten Alkyl- und Alkoxyteile jeweils 1 bis 3 Kohlenstoffatome und die Cycloalkylteile jeweils 5 oder 6 Kohlenstoffatome enthalten können,
deren Stereoisomere, deren Gemische und deren Salze.

Besonders bevorzugte Verbindungen der obigen allgemeinen Formel I sind jedoch diejenigen, in denen
einer der Reste $R_a$ oder $R_b$ eine A-B-Gruppe und
der andere der Reste $R_a$ oder $R_b$ eine F-E-D-Gruppe, in denen

A eine Aminoalkylgruppe mit 1 bis 4 Kohlenstoffatomen im Alkylteil, eine Amino- oder Amidinogruppe, wobei in den vorstehend erwähnten Amino-, Aminoalkyl- oder Amidinogruppen eine Amino- oder Iminogruppe durch eine Methylgruppe oder durch eine in vivo abspaltbare Gruppe substituiert sein kann,

B eine Phenylengruppe, die durch eine Methylgruppe, durch ein Fluor-, Chlor- oder Bromatom substituiert sein kann, eine Pyridinylen-, 1,3-Piperidinylen- oder 1,4-Piperidinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf,

eine Cyclohexylengruppe,

eine Indanyliden- oder 1,2,3,4-Tetrahydronaphthylidengruppe, wobei jeweils der aromatische Kern mit dem cyclischen Iminorest verknüpft ist, oder

A und B zusammen eine Piperidinylgruppe,

D eine Phenylengruppe, die durch ein Fluor-, Chlor- oder Bromatom, durch eine Carboxymethoxy- oder Methosycarbonylmethoxygruppe substituiert sein kann,

eine Cyclohexylen-, Pyridinylen-, Pyrimidinylen-, 1,3-Piperidinylen- oder 1,4-Piperidinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf,

E eine gegebenenfalls durch eine Hydroxy-, Methoxy-, Amino-, Acetylamino- oder Methansulfonylaminogruppe substituierte geradkettige oder verzweigte Alkylengruppe mit 1 bis 4 Kohlenstoffatomen, wobei in einer Alkylengruppe mit 2 oder 3 Kohlenstoffatomen die mit dem Rest D verknüpfte Methylengruppe durch ein Sauerstoff- oder Schwefelatom, durch eine Sulfinyl-, Sulfonyl-, Imino-, Methylimino-, Carboxymethylimino- oder Methoxycarbonylmethyliminogruppe ersetzt sein kann, oder eine Alkenylengruppe mit 2 oder 3 Kohlenstoffatomen und

F eine in vivo zu einer Carboxylgruppe metabolisierbare Gruppe, eine Carboxylgruppe, eine Phosphono-, O-Methylphosphono- oder Tetrazol-5-ylgruppe darstellen, und entweder

(a) X eine Carbonylgruppe,
$R_d$ und $R_e$ zusammen eine weitere Kohlenstoff-Kohlenstoff-Bindung,
$R_c$ ein Wasserstoffatom, eine Phenylgruppe, eine Hydroxygruppe, eine geradkettige oder verzweigte Alkoxygruppe mit 1 bis 3 Kohlenstoffatomen, eine Phenylmethoxy-, Morpholinocarbonylmethoxy-, Methylamino-, Dimethylamino-, Phenylamino-, Pyrrolidino- oder Piperidinogruppe, wobei die Methylengruppe in 4-Stellung einer Piperidinogruppe durch ein Sauerstoff- oder Schwefelatom, durch eine

Sulfinyl-, Sulfonyl-, Imino- oder Acetyliminogruppe ersetzt sein kann,
$R_f$ ein Wasserstoffatom, eine Methylgruppe oder eine Phenylgruppe und
$R_g$ ein Wasserstoffatom oder eine Methylgruppe oder
(b) X eine Carbonylgruppe,
$R_c$ und $R_e$ zusammen mit dem dazwischen liegenden Kohlenstoffatom eine Carbonylgruppe,
$R_d$ eine Methyl-, Phenylmethyl- oder Morpholinocarbonylmethylgruppe,
$R_f$ und $R_g$ jeweils ein Wasserstoffatom oder
(c) X eine Methylengruppe,
$R_f$ und $R_g$ zusammen mit dem dazwischen liegenden Kohlenstoffatom eine Carbonylgruppe,
$R_c$, $R_d$ und $R_e$ jeweils ein Wasserstoffatom oder
$R_c$ ein Wasserstoffatom oder auch, wenn B oder D eine 1,3-Piperidinylen- oder 1,4-Piperidinylengruppe, welche über die Position 1 mit dem cyclischen Iminorest verknüpft sind, darstellt, eine Phenylgruppe und
$R_d$ und $R_e$ zusammen eine weitere Kohlenstoff-Kohlenstoff-Bindung oder
(d) X eine Methylen- oder Carbonylgruppe und
$R_c$ bis $R_f$ jeweils ein Wasserstoffatom bedeuten,
wobei unter "eine in vivo zu einer Carboxylgruppe metabolisierbare Gruppe" und unter "eine in vivo abspaltbare Gruppe" beispielsweise eine Estergruppe der Formeln

- CO - OR',
- CO - O - (HCR") - O - CO - R''' und
- CO - O - (HCR") - O - CO - OR''',

in denen
R' eine geradkettige oder verzweigte Alkylgruppe mit 1 bis 3 Kohlenstoffatomen, wobei die Methylgruppe durch eine Dimethylaminocarbonyl-, Piperidinocarbonyl- oder Morpholinocarbonylgruppe substituiert sein kann, eine Allyl-, Phenylmethyl-, 2-Morpholinoethyl- oder Cyclohexylgruppe,
R" ein Wasserstoffatom oder eine Methylgruppe und
R''' eine geradkettige oder verzweigte Alkylgruppe mit 1 bis 4 Kohlenstoffatomen oder eine Cyclohexylgruppe bedeuten, zu verstehen ist, wobei eine nach der Abspaltung so gebildete Carboxylgruppe, falls diese an ein Stickstoffatom des Restes A gebunden ist, unter Freisetzung der Amino- oder Iminogruppe decarboxyliert werden kann, bedeuten,
deren Stereoisomere, deren Gemische und deren Salze.

Ganz besonders bevorzugte Verbindungen der allgemeinen Formel I sind jedoch diejenigen, in denen einer der Reste $R_a$ oder $R_b$ eine A-B-Gruppe und der andere der Reste $R_a$ oder $R_b$ eine F-E-D-Gruppe, in denen

A eine Aminoalkylgruppe mit 1 bis 4 Kohlenstoffatomen im Alkylteil, eine Amino- oder Amidinogruppe, wobei in den vorstehend erwähnten Amino-, Aminoalkyl- oder Amidinogruppen eine Amino- oder Iminogruppe durch eine Methylgruppe oder durch eine in vivo abspaltbare Gruppe substituiert sein kann,

B eine Phenylengruppe, die durch eine Methylgruppe, durch ein Fluor-, Chlor- oder Bromatom substituiert sein kann, eine Pyridinylen-, 1,3-Piperidinylen- oder 1,4-Piperidinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf,

eine Cyclohexylengruppe,

eine Indanyliden- oder 1,2,3,4-Tetrahydronaphthylidengruppe, wobei jeweils der aromatische Kern mit dem cyclischen Iminorest verknüpft ist, oder

A und B zusammen eine Piperidinylgruppe,

D eine Phenylengruppe, die durch ein Fluor-, Chlor- oder Bromatom, durch eine Carboxymethoxy- oder Methoxycarbonylmethoxygruppe substituiert sein kann,

eine Cyclohexylen-, Pyridinylen-, Pyrimidinylen-, 1,3-Piperidinylen- oder 1,4-Piperidinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf,

E eine gegebenenfalls durch eine Hydroxy-, Methoxy- oder Aminogruppe substituierte geradkettige oder verzweigte Alkylengruppe mit 1 bis 4 Kohlenstoffatomen, wobei in einer Alkylengruppe mit 2 oder 3 Kohlenstoffatomen die mit dem Rest D verknüpfte Methylengruppe durch ein Sauerstoff- oder Schwefelatom, durch eine Sulfinyl-, Sulfonyl-, Imino-, Methylimino-, Carboxymethylimino- oder Methoxycarbonylmethyliminogruppe ersetzt sein kann, oder eine Alkenylengruppe mit 2 oder 3 Kohlenstoffatomen und

F eine in vivo zu einer Carboxylgruppe metabolisierbare Gruppe, eine Carboxylgruppe, eine Phosphono-, O-Methylphosphono- oder Tetrazol-5-ylgruppe darstellen, und entweder

(a) X eine Carbonylgruppe,
$R_d$ und $R_e$ zusammen eine weitere Kohlenstoff-Kohlenstoff-Bindung,
$R_c$ ein Wasserstoffatom, eine Phenylgruppe, eine Hydroxygruppe, eine geradkettige oder verzweigte Alkoxygruppe mit 1 bis 3 Kohlenstoffatomen, eine Phenylmethoxy-, Morpholinocarbonylmethoxy-, Methylamino-, Dimethylamino-, Phenylamino-, Pyrrolidino- oder Piperidinogruppe, wobei die Methylengruppe in 4-Stellung einer Piperidinogruppe durch ein Sauerstoff- oder Schwefelatom, durch eine Sulfinyl-, Sulfonyl-, Imino- oder Acetyliminogruppe ersetzt sein kann,
$R_f$ ein Wasserstoffatom, eine Methylgruppe oder eine Phenylgruppe und
$R_g$ ein Wasserstoffatom oder eine Methylgruppe oder
(b) X eine Carbonylgruppe,
$R_c$ und $R_e$ zusammen mit dem dazwischen liegenden Kohlenstoffatom eine Carbonylgruppe,
$R_d$ eine Methyl-, Phenylmethyl- oder Morpholinocarbonylmethylgruppe,
$R_f$ und $R_g$ jeweils ein Wasserstoffatom oder
(d) X eine Carbonylgruppe und
$R_c$ bis $R_f$ jeweils ein Wasserstoffatom bedeuten,
wobei unter "eine in vivo zu einer Carboxylgruppe metabolisierbare Gruppe" und unter "eine in vivo abspaltbare Gruppe" beispielsweise eine Estergruppe der Formeln

- CO - OR',
- CO - O - (HCR'') - O - CO - R''' und
- CO - O - (HCR'') - O - CO - OR''',

in denen
R' eine geradkettige oder verzweigte Alkylgruppe mit 1 bis 3 Kohlenstoffatomen, wobei die Methylgruppe durch eine Dimethylaminocarbonyl-, Piperidinocarbonyl- oder Morpholinocarbonylgruppe substituiert sein kann, eine Allyl-, Phenylmethyl-, 2-Morpholinoethyl- oder Cyclohexylgruppe,
R'' ein Wasserstoffatom oder eine Methylgruppe und
R''' eine geradkettige oder verzweigte Alkylgruppe mit 1 bis 4 Kohlenstoffatomen oder eine Cyclohexylgruppe bedeuten, zu verstehen ist, wobei eine nach der Abspaltung so gebildete Carboxylgruppe, falls diese an ein Stickstoffatom des Restes A gebunden ist, unter Freisetzung der Amino- oder Iminogruppe decarboxyliert werden kann, bedeuten,
insbesondere diejenigen Verbindungen der obigen allgemeinen Fomel I, in der
einer der Reste $R_a$ oder $R_b$ eine A-B-Gruppe und
der andere der Reste $R_a$ oder $R_b$ eine F-E-D-Gruppe, in denen
A eine Aminomethyl- oder Amidinogruppe,
B eine Phenylengruppe,
D eine Phenylengruppe,
E eine Ethylengruppe und
F eine in vivo zu einer Carboxylgruppe metabolisierbare Gruppe oder eine Carboxylgruppe darstellen, und entweder
(a) X eine Carbonylgruppe,
$R_d$ und $R_e$ zusammen eine weitere Kohlenstoff-Kohlenstoff-Bindung,
$R_c$ eine Hydroxy-, Methoxy-, Ethoxy- oder Morpholinogruppe,
$R_f$ und $R_g$ jeweils ein Wasserstoffatom oder
(b) X eine Carbonylgruppe,
$R_c$ und $R_e$ zusammen mit dem dazwischen liegenden Kohlenstoffatom eine Carbonylgruppe,
$R_d$ eine Methylgruppe,
$R_f$ und $R_g$ jeweils ein Wasserstoffatom oder
(d) X eine Carbonylgruppe und
$R_c$ bis $R_f$ jeweils ein Wasserstoffatom bedeuten,
wobei unter "eine in vivo zu einer Carboxylgruppe metabolisierbare Gruppe" eine R'O-CO-Gruppe, in der R' eine geradkettige oder verzweigte Alkylgruppe mit 1 bis 3 Kohlenstoffatomen darstellt, zu verstehen ist, bedeuten,
deren Stereoisomere, deren Gemische und deren Salze.

Als besonders bevorzugte Verbindungen seien beispielsweise folgende genannt:
(a) 1-(4-Amidinophenyl)-3-[4-(2-carboxy-ethyl)-phenyl]-4-methoxy-3-pyrrolin-2-on,
(b) 1-(4-Amidinophenyl)-3-[4-(2-carboxy-ethyl)-phenyl]-4-morpholino-3-pyrrolin-2-on,
(c) 1-(4-Amidinophenyl)-3-[4-(2-carboxy-ethyl)-phenyl]-pyrrolidin-2-on,

(d) 1-(4-Aminomethyl-phenyl)-3-[4-(2-carboxy-ethyl)-phenyl]-4-methoxy-3-pyrrolin-2-on und

(e) 1-(4-Amidinophenyl)-3-[4-(2-carboxy-ethyl)-phenyl]-3-methyl-pyrrolidin-2,4-dion,

deren Stereoisomere, deren Gemische und deren Salze.

Die neuen Verbindungen lassen sich beispielsweise nach folgenden Verfahren herstellen:

a) Zur Herstellung von Verbindungen der allgemeinen Formel I, in der F eine Carboxylgruppe darstellt: Umwandlung einer Verbindung der allgemeinen Formel

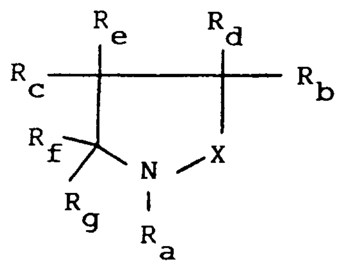

,(II)

in der

X und $R_a$ bis $R_g$ mit der Maßgabe wie eingangs definiert sind, daß

einer der Reste $R_a$ oder $R_b$ eine A-B-Gruppe und

der andere der Reste $R_a$ oder $R_b$ eine F'-E-D-Gruppe bedeutet, wobei B, E und D wie eingangs definiert sind und F' eine mittels Hydrolyse, Behandeln mit Säuren, Thermolyse oder Hydrogenolyse in eine Carboxylgruppe überführbare Gruppe bedeutet, in eine Verbindung der allgemeinen Formel I, in der F eine Carboxylgruppe darstellt.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe,

Ester mit tertiären Alkoholen, z.B. der tert. Butylester, mittels Behandlung mit einer Säure oder Thermolyse in eine Carboxylgruppe und

Ester mit Aralkanolen, z.B. der Benzylester, mittels Hydrogenolyse in eine Carboxylgruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure, in Gegenwart einer Base wie Lithiumhydroxid, Natriumhydroxid oder Kaliumhydroxid in einem geeigneten Lösungsmittel wie Wasser, Wasser/Methanol, Wasser/Äthanol, Wasser/Isopropanol, Methanol, Äthanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt. Bei der Behandlung mit einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet F' in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C in die Carboxylgruppe übergeführt werden.

Bedeutet F' in einer Verbindung der Formel II beispielsweise die tert. Butyloxycarbonylgruppe, so kann die tert. Butylgruppe auch durch Behandlung mit einer Säure wie Trifluoressigsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 100°C, abgespalten werden.

Bedeutet F' in einer Verbindung der Formel II beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig,

Essigsäureäthylester, Dioxan oder Dimethylformamid vorzugsweise bei Temperaturen zwischen 0 und 50 °C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Methoxygruppe über eine $\beta$-Eliminierung zu einem Wasserstoffatom oder eine ungesättigte Verbindung zu einer gesättigten Verbindung mitreduziert werden.

b) Zur Herstellung von Verbindungen der allgemeinen Formel I, in der A eine gegebenenfalls an der Aminogruppe durch eine oder zwei Alkylgruppen substituierte $H_2N-C(=NH)$-Gruppe darstellt:
Umsetzung einer gegebenenfalls im Reaktionsgemisch gebildeten Verbindung der allgemeinen Formel

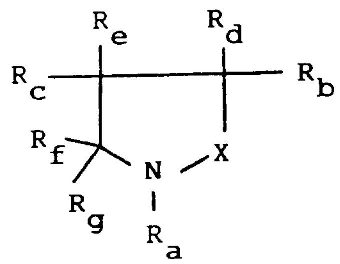

,(III)

in der
X und $R_a$ bis $R_g$ mit der Maßgabe wie eingangs definiert sind, daß
einer der Reste $R_a$ oder $R_b$ eine NC-B-Gruppe und
der andere der Reste $R_a$ oder $R_b$ eine F-E-D-Gruppe bedeutet, wobei B, E und D wie eingangs definiert sind, mit einem Amin der allgemeinen Formel

$(R_1')_2NH$ ,(IV)

in der
die Reste $R_1'$, die gleich oder verschieden sein können, Wasserstoffatome oder Alkylgruppen mit jeweils 1 bis 4 Kohlenstoffatomen darstellen, oder mit deren Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150 °C, vorzugsweise bei Temperaturen zwischen 20 und 120 °C, mit einem entsprechenden freien Amin oder mit einem entsprechenden Säureadditionssalz wie beispielsweise den entsprechenden Ammoniumcarbonaten, -acetaten oder -chloriden durchgeführt.

Eine Verbindung der allgemeinen Formel III erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen 0 und 50 °C, vorzugsweise jedoch bei 20 °C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid oder durch Umsetzung eines entsprechenden Nitrils mit einem Alkoholat wie Natriummethylat in einem Lösungsmittel wie Dioxan oder Tetrahydrofuran, vorzugsweise jedoch in dem betreffenden Alkohol.

c) Zur Herstellung von Verbindungen der allgemeinen Formel I, in der mindestens einer der Reste $R_a$ bis $R_g$ eine Sulfinyl- oder Sulfonylgruppe enthält:
Oxidation einer Verbindung der allgemeinen Formel

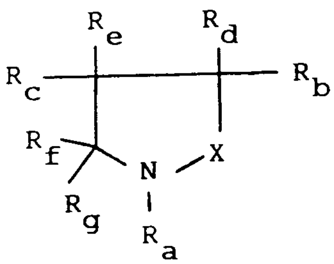

,(V)

in der
X und $R_a$ bis $R_g$ mit der Maßgabe wie eingangs definiert sind, daß mindestens einer der Reste $R_a$ bis $R_g$ eine Sulfenyl- oder Sulfinylgruppe enthält.

Die Oxidation wird vorzugsweise in einem Lösungsmittel oder Lösungsmittelgemisch, z.B. in Wasser, Wasser/Pyridin, Aceton, Methylenchlorid, Eisessig, Eisessig/Acetanhydrid, verdünnter Schwefelsäure oder Trifluoressigsäure, je nach dem verwendeten Oxidationsmittel zweckmäßigerweise bei Temperaturen zwischen -80 und 100 °C durchgeführt.

Zur Herstellung einer entsprechenden S-Oxidverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20 °C oder in Aceton bei 0 bis 60 °C, mit einer Persäure wie Perameisensäure in Eisessig oder Trifluoressigsäure bei 0 bis 50 °C oder mit m-Chlorperbenzoesäure in Methylenchlorid oder Chloroform bei -20 bis 60 °C, mit Natriummetaperjodat in wäßrigem Methanol oder Äthanol bei -15 bis 25 °C, mit Brom in Eisessig oder wäßriger Essigsäure gegebenenfalls in Gegenwart einer schwachen Base wie Natriumacetat, mit N-Brom-succinimid in Äthanol, mit tert.Butyl-hypochlorit in Methanol bei -80 bis -30 °C, mit Jodbenzoldichlorid in wäßrigem Pyridin bei 0 bis 50 °C, mit Salpetersäure in Eisessig bei 0 bis 20 °C, mit Chromsäure in Eisessig oder in Aceton bei 0 bis 20 °C und mit Sulfurylchlorid in Methylenchlorid bei -70 °C, der hierbei erhaltene Thioäther-Chlor-Komplex wird zweckmäßigerweise mit wäßrigem Äthanol hydrolysiert.

Zur Herstellung einer S,S-Dioxidverbindung der allgemeinen Formel I wird die Oxidation ausgehend von einer entsprechenden Alkylsulfinylverbindung zweckmäßigerweise mit einem oder mehr Äquivalenten des verwendeten Oxidationsmittels oder ausgehend von einer entsprechenden Alkylsulfenylverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100 °C oder in Aceton bei 0 bis 60 °C, mit einer Persäure wie Perameisensäure oder m-Chlorperbenzoesäure in Eisessig, Trifluoressigsäure, Methylenchlorid oder Chloroform bei Temperaturen zwischen 0 und 60 °C, mit Salpetersäure in Eisessig bei 0 bis 20 °C, mit Chromsäure oder Kaliumpermanganat in Eisessig, Wasser/Schwefelsäure oder in Aceton bei 0 bis 20 °C.

d) Zur Herstellung von Verbindungen der allgemeinen Formel I, in der A eine Aminogruppe, eine Aminoalkylgruppe mit 1 bis 4 Kohlenstoffatomen im Alkylteil, eine Amidino- oder Guanidinogruppe darstellt, wobei in den vorstehend erwähnten Gruppen die Amino- oder Iminogruppe durch eine Alkylgruppe mit 1 bis 4 Kohlenstoffatomen oder durch eine Phenylalkylgruppe substituiert sein kann, die alle zusätzlich am Stickstoffatom durch eine in vivo abspaltbare Gruppe substituiert sind:
Umsetzung einer Verbindung der allgemeinen Formel

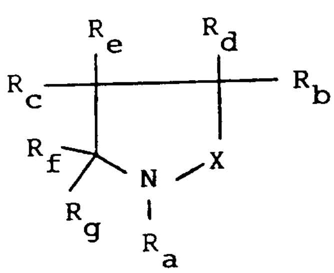

,(VI)

in der
X und $R_a$ bis $R_g$ mit der Maßgabe wie eingangs definiert sind, daß
einer der Reste $R_a$ oder $R_b$ eine A'-B-Gruppe und

der andere der Reste $R_a$ oder $R_b$ eine F-E-D-Gruppe bedeutet, wobei B, D, E und F wie eingangs definiert sind und A' eine Aminogruppe, eine Aminoalkylgruppe mit 1 bis 4 Kohlenstoffatomen im Alkylteil, eine Amidino- oder Guanidinogruppe darstellt, wobei in den vorstehend erwähnten Gruppen die Amino- oder Iminogruppe durch eine Alkylgruppe mit 1 bis 4 Kohlenstoffatomen oder durch eine Phenylalkylgruppe substituiert sein kann, mit einer Verbindung der allgemeinen Formel

$Z_1$ - $R_3$ ,(VII)

in der
$R_3$ eine Gruppe der Formeln

- CO - OR',
- CO - O - (HCR'') - O - CO - R''' und
- CO - O - (HCR'') - O - CO - OR''' darstellt, in denen

R', R'' und R''' wie eingangs definiert sind, und
$Z_1$ eine nukleophile Austrittsgruppe wie ein Halogenatom,
z. B. ein Chlor- oder Bromatom, oder eine gegebenenfalls substituierte Phenoxygruppe, z. B. die p-Nitrophenyloxygruppe, darstellt.

Die Umsetzung wird zweckmäßigerweise in einem Lösungsmittel wie Tetrahydrofuran, Methylenchlorid, Chloroform oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100 °C, vorzugsweise jedoch bei Temperaturen zwischen -10 und 80 °C, durchgeführt.

e) Zur Herstellung von Verbindungen der allgemeinen Formel I, in der A eine Aminoalkylgruppe darstellt:
Reduktion einer Verbindung der allgemeinen Formel

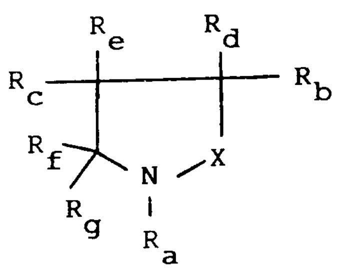

,(VIII)

in der X und $R_a$ bis $R_g$ mit der Maßgabe wie eingangs definiert sind, daß
einer der Reste $R_a$ oder $R_b$ eine A''-B-Gruppe und
der andere der Reste $R_a$ oder $R_b$ eine F-E-D-Gruppe bedeutet, wobei B, D, E und F wie eingangs definiert sind und A'' eine Cyano- oder Cyanoalkylgruppe darstellt.

Die Reduktion wird vorzugsweise in einem geeigneten Lösungsmittel wie Methanol, Methanol/Wasser, Methanol/Wasser/Ammoniak, Methanol/Ammoniak, Methanol/Salzsäure, Eisessig, Ethanol, Ether, Tetrahydrofuran, Dioxan oder Dimethylformamid in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100 °C, vorzugsweise bei Temperaturen zwischen 20 und 80 °C, durchgeführt. Bei der Reduktion können gleichzeitig andere Reste, z.B. eine Nitrogruppe zur Aminogruppe, eine Benzyloxygruppe zur Hydroxygruppe, eine Methoxygruppe über eine $\beta$-Eliminierung zu einem Wasserstoffatom oder eine ungesättigte Verbindung zu einer gesättigten Verbindung mitreduziert werden.

f) Zur Herstellung von Verbindungen der allgemeinen Formel I, in der A eine Amidinogruppe und B eine Cycloalkyleniminogruppe mit 4 bis 6 Kohlenstoffatomen darstellen, wobei die Amidinogruppe mit dem Stickstoffatom der Cycloalkyleniminogruppe verknüpft ist:
Umsetzung einer Verbindung der allgemeinen Formel

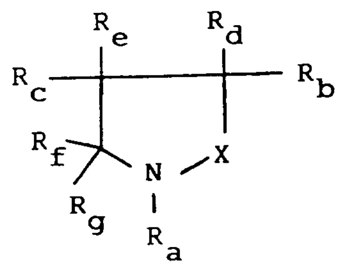

,(IX)

in der
X und $R_a$ bis $R_g$ mit der Maßgabe wie eingangs definiert sind, daß
einer der Reste $R_a$ oder $R_b$ eine H-B"-Gruppe und
der andere der Reste $R_a$ oder $R_b$ eine F-E-D-Gruppe bedeutet, wobei D, E und F wie eingangs definiert sind und
B" eine Cycloalkyleniminogruppe mit 4 bis 6 Kohlenstoffatomen darstellt, mit einem S-Alkyl-isothioharnstoff, in dem der Alkylteil zweckmäßigerweise 1 bis 3 Kohlenstoffatome enthalten kann.

Die Umsetzung wird zweckmäßigerweise in einem Lösungsmittel wie Dimethylformamid und vorzugsweise in Gegenwart einer Base wie Natriumcarbonat bei erhöhten Temperaturen, z.B. bei Temperaturen zwischen 80 und 120 °C, durchgeführt.

g) Zur Herstellung der Verbindungen der allgemeinen Formel I, in der $R_e$ und $R_d$ jeweils ein Wasserstoffatom darstellen:
Hydrierung einer Verbindung der allgemeinen Formel

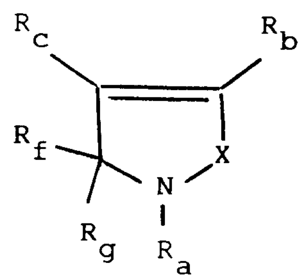

,(X)

in der
X, $R_a$ bis $R_c$, $R_f$ und $R_g$ wie eingangs definiert sind.

Die Hydrierung wird vorzugsweise in einem geeigneten Lösungsmittel wie Methanol, Methanol/Wasser, Methanol/Wasser/Ammoniak, Methanol/Ammoniak, Methanol/Salzsäure, Eisessig, Ethanol, Ether, Tetrahydrofuran, Dioxan oder Dimethylformamid in Gegenwart von katalytisch angeregtem Wasserstoff, z.B. von Wasserstoff in Gegenwart von Raney-Nickel, Platin oder Palladium/Kohle, bei Temperaturen zwischen 0 und 100 °C, vorzugsweise bei Temperaturen zwischen 20 und 80 °C, durchgeführt. Bei der Hydrierung können gleichzeitig andere Reste, z.B. eine Nitrogruppe zur Aminogruppe, eine Benzyloxygruppe zur Hydroxygruppe, eine Methoxygruppe über eine $\beta$-Eliminierung zu einem Wasserstoffatom oder eine ungesättigte Verbindung zu einer gesättigten Verbindung mitreduziert werden.

h) Zur Herstellung von Verbindungen der allgemeinen Formel I, in der F eine R'O-CO-, R'''-CO-O-(HCR")-O-CO- oder R'''O-CO-O-(HCR")-O-CO-Gruppe darstellt:
Veresterung einer Verbindung der allgemeinen Formel

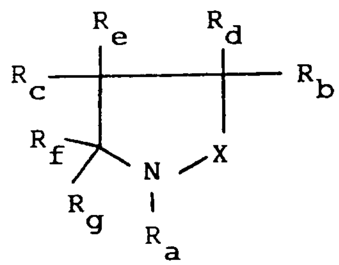

,(XI)

in der
X und $R_a$ bis $R_g$ mit der Maßgabe wie eingangs definiert sind, daß F eine Carboxygruppe darstellt, oder deren reaktionsfähige Derivate mit einer Verbindung der allgemeinen Formel

$Z_2$ - $R_4$ ,(XII)

in der
$R_4$ eine R'-, R'''-CO-O-(HCR'')- oder R'''O-CO-O-(HCR'')-Gruppe, wobei R', R'' und R''' wie eingangs definiert sind, und
$Z_2$ eine Hydroxygruppe oder eine nukleophile Austrittsgruppe wie ein Halogenatom, z. B. ein Chlor-, Brom- oder Jodatom, darstellt.

Die Veresterung wird zweckmäßigerweise in einem geeigneten Lösungsmittel, z. B. in einem entsprechenden Alkohol wie Methanol, Ethanol oder Isopropanol, Methylenchlorid, Tetrahydrofuran, Dioxan, Pyridin, Toluol oder Dimethylsulfoxid in Gegenwart eines säureaktivierenden und/oder wasserentziehenden Mittels wie Chlorwasserstoff, konz. Schwefelsäure, Thionylchlorid, Chlorameisensäureethylester, Carbonyldiimidazol oder N,N'-Dicyclohexyl-carbodiimid oder dessen Isoharnstoffestern, gegebenenfalls in Gegenwart eines Reaktionsbeschleunigers wie Kupferchlorid, oder durch Umesterung, z. B. mit einem entsprechenden Kohlensäurediester, durchgeführt.

Die Umsetzung mit einem entsprechenden Halogenid, vorzugsweise mit einem Halogenid, in dem $R_4$ eine R'''-CO-O-(HCR'')- oder R'''O-CO-O-(HCR'')-Gruppe darstellt, wird vorzugsweise in Gegenwart einer Base wie Triethylamin und gegebenenfalls in Gegenwart eines Reaktionsbeschleunigers wie Kaliumjodid bei Temperaturen zwischen 0 und 100 °C, vorzugsweise jedoch bei Temperaturen zwischen 20 °C und der Siedetemperatur des betreffenden Lösungsmittels, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Benzoyl-, Ethoxycarbonyl-, tert.Butoxycarbonyl-, Benzyloxycarbonyl-, Allyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsaure oder Schwefelsäure oder in Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 100 °C, vorzugsweise bei Temperaturen zwischen 10 und 50 °C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50 °C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100 °C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70 °C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50 °C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem stereogenem Zentrum in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 stereogenen Zentren auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihren aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Essigsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Ammoniak, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren wie diese beispielsweise in den Beispielen I bis VII beschrieben werden. So erhält man die als Ausgangsstoffe verwendeten Verbindungen der allgemeinen Formeln II, III, V, VI und VIII bis XI durch Ringschluß eines entsprechend substituierten Arylessigsäureamids, das am Säureamidstickstoffatom durch einen entsprechenden β-Ketorest substituiert ist. Ein so erhaltenes entsprechend substituiertes 4-Hydroxy-3-pyrrolidin-2-on kann anschließend mittels Alkylierung oder durch Umsetzung mit einem entsprechenden Amin in die gewünschte Verbindung übergeführt werden. Das hierfür erforderliche Essigsäureamid erhält man beispielsweise durch Umsetzung eines entsprechenden Arylessigsäureamids mit einem entsprechenden α-Halogen-essigsäurederivat.

Wie bereits eingangs erwähnt, weisen die neuen 5-gliedrigen Heterocyclen der allgemeinen Formel I und deren Salze, insbesondere deren physiologisch verträgliche Salze mit anorganischen oder organischen Säuren oder Basen, wertvolle pharmakologische Eigenschaften auf. So weisen die neuen Verbindungen neben einer entzündungshemmenden und den Knochenabbau hemmenden Wirkung insbesondere antithrombotische, antiaggregatorische und tumor- bzw. metastasenhemmende Wirkungen auf.

Beispielsweise wurden die Verbindungen der allgemeinen Formel I auf ihre biologischen Wirkungen wie folgt untersucht:

1. Hemmung der Bindung von $^{3}$H-BIBU 52 an Humanthrombozyten:

Eine Suspension von Humanthrombozyten in Plasma wird mit $^{3}$H-BIBU 52 [= (3S,5S)-5-[(4'-Amidino-4-biphenylyl)oxymethyl]-3-[(carboxyl)methyl]-2-pyrrolidinon[3-$^{3}$H-4-biphenylyl]], das den literaturbekannten Liganden $^{125}$J-Fibrinogen ersetzt, (siehe deutsche Patentanmeldung P 42 14 245.8 der gleichen Anmelderin vom 30. 04. 1992, internes Zeichen: Case 5/1093-FL) und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^{3}$H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma nocheinmal scharf abzentrifugiert. Das PRP wird mit autologem Plasma 1:10 verdünnt. 750 µl werden mit 50 µl physiologischer Kochsalzlösung, 100 µl Testsubstanzlösung, 50 µl $^{14}$C-Sucrose (3.700 Bq) und 50 µl $^{3}$H-BIBU 52 (Endkonzentration: 5 nM) bei Raumtemperatur 20 Minuten inkubiert. Zur Messung der unspezifischen Bindung wird anstelle der Testsubstanz 5 µl BIBU 52 (Endkonzentration: 30 µM) eingesetzt. Die Proben werden 20 Sekunden bei 10000 x g zentrifugiert und der Überstand abgezogen. 100 µl hiervon werden zur Bestimmung des freien Liganden gemessen. Das Pellet wird in 500 µl 0,2N NaOH gelöst, 450 µl werden mit 2 ml Szintillator und 25 µl 5N HCl versetzt und gemessen. Das im Pellet noch verbliebene Restplasma wird aus dem $^{14}$C-Gehalt bestimmt, der gebundene Ligand aus der $^{3}$H-Messung. Nach Abzug der unspezifischen Bindung wird die Pelletaktivität gegen die Konzentration der Testsubstanz aufgetragen und die Konzentration für eine 50%ige Hemmung der Bindung ermittelt.

2. Antithrombotische Wirkung:

Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37°C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[nM] | Hemmung der Plättchenaggregation $EC_{50}$[nM] |
|---|---|---|
| 2 | 11 | 40 |
| 2(3) | 42 | 730 |
| 2(13) | 110 | 260 |
| 2(55) | 180 | 330 |
| 5(B) | 8.600 | 380 |
| 13 | 360 | 500 |

Die erfindungsgemäßen Verbindungen sind gut verträglich, da beispielsweise nach intravenöser Gabe von 15 mg/kg der Verbindung des Beispiels 2 an der Maus keines der 3 getesteten Tiere verstarb.

Auf Grund ihrer Hemmwirkung auf Zell-Zell- bzw. Zell-Matrix-Wechselwirkungen eignen sich die neuen cyclischen Iminoderivate der allgemeinen Formel I und ihre physiologisch verträglichen Salze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 $\mu$g und 20 mg/kg Körpergewicht, vorzugsweise bei 1 $\mu$g bis 10 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, $\alpha$-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanze wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Herstellung der Ausgangsverbindungen:

Beispiel I

p-(Carboxymethyl)-zimtsäure-methylester

Unter Inertgas wird eine Lösung von 32,3 g 4-Bromphenylessigsäure, 21 g Acrylsäure-methylester, 44 g Triethylamin, 2,5 g Tri-o-tolylphosphin und 0,5 g Palladium(II)acetat 4,5 Stunden zum Rückfluß erhitzt. Das abgekühlte Reaktionsgemisch wird in 600 ml eisgekühlte, verdünnte Salzsäure eingerührt. Die wässrige Phase wird dreimal mit Essigester extrahiert, die organische Phase über Natriumsulfat getrocknet und das Lösungsmittel abdestilliert. Der verbleibende Feststoff wird mit Essigester verrieben und abgenutscht.
Ausbeute: 28,5 g (86 % der Theorie),
Schmelzpunkt: 120-126 °C
$R_f$-Wert: 0,40 (Kieselgel; Methylenchlorid/Methanol = 9:1)

Beispiel II

3-(4-Carboxymethyl-phenyl)-propionsäure-methylester

18,0 g p-(Carboxymethyl)-zimtsäure-methylester in 900 ml Eisessig werden 2 Stunden bei Raumtemperatur unter einem Wasserstoffdruck von 5 bar in Gegenwart von 2 g 10 % Palladium auf Aktivkohle hydriert. Der Katalysator wird anschließend abfiltriert und das Filtrat eingedampft. Der erhaltene Feststoff wird mit Wasser verrieben und abgesaugt.
Ausbeute: 17,5 g (96 % der Theorie),
Schmelzpunkt: 101-104 °C
$R_f$-Wert: 0,12 (Kieselgel; Methylenchlorid/Methanol = 20:1)

Beispiel III

3-[4-[[(4-Cyanophenyl)-aminocarbonyl]-methyl]-phenyl]-propionsäure-methylester

Unter Inertgas tropft man bei -20 °C 44,5 g Diphenylphosphinsäurechlorid zu einer Lösung von 33,0 g 3-(4-Carboxymethylphenyl)-propionsäure-methylester und 15,7 g Triethylamin in 250 ml wasserfreiem Tetrahydrofuran. Man rührt 60 Minuten bei -20 °C und tropft bei dieser Temperatur eine Lösung von 17,72 g 4-Aminobenzonitril und 18,3 g Dimethylaminopyridin in 250 ml wasserfreiem Tetrahydrofuran zu. Man rührt 30 Minuten bei -20 °C, entfernt das Kühlbad und rührt weitere 16 Stunden bei Raumtemperatur. Die Suspension wird mit 500 ml Essigsäure-ethylester verdünnt und die Lösung einmal mit 1N Salzsäure und einmal mit Wasser gewaschen. Die organische Phase wird über Natriumsulfat getrocknet, das Lösungsmittel eingedampft und der verbleibende Rückstand mit Methylenchlorid/Methanol (20:1) über Kieselgel chromatographiert.
Ausbeute: 19,5 g (41 % der Theorie),
Schmelzpunkt: 146-148 °C $R_f$-Wert: 0,18 (Kieselgel; Methylenchlorid/Methanol = 20:1)

Beispiel IV

3-[4-[[N-(4-Cyanophenyl)-N-[(methoxycarbonyl)-methyl]-aminocarbonyl]-methyl]-phenyl]-propionsäure-methylester

Eine Lösung von 14,5 g 3-[4-[[(4-Cyanophenyl)-aminocarbonyl]-methyl]-phenyl]-propionsäure-methylester in 100 ml wasserfreiem Dimethylformamid versetzt man unter Inertgas bei -40 °C portionsweise mit 1,96 g einer 55%igen Dispersion von Natriumhydrid in Mineralöl. Man entfernt das Kühlbad und rührt solange bis das Natriumhydrid vollständig gelöst ist. Anschließend tropft man bei -30 °C 23,0 g Bromessigsäure-methylester zu und rührt 30 Minuten bei dieser Temperatur. Das Kühlbad wird entfernt und 16 Stunden bei Raumtemperatur gerührt. Die Reaktionslösung wird auf 500 ml 1N Salzsäure gegossen und die wässrige Phase dreimal mit Essigsäure-ethylester extrahiert. Die organische Phase wird mit Wasser gewaschen, über Natriumsulfat getrocknet und das Lösungsmittel eingedampft. Der Rückstand wird mit Methylenchlorid/Methanol (9:1) über Kieselgel chromatographiert. Man erhält 11,0 g Produkt, welches mit Ausgangsmaterial verunreinigt ist und ohne weitere Reinigung in Beispiel V eingesetzt wird.
$R_f$-Wert: 0,71 (Kieselgel; Methylenchlorid/Methanol = 9:1)

Beispiel V

1-(4-Cyanophenyl)-4-hydroxy-3-[4-(2-methoxycarbonyl-ethyl)-phenyl]-3-pyrrolin-2-on

Zu einer Lösung von 11,0 g 3-[4-[[N-(4-Cyanophenyl)-N-[(meth-oxycarbonyl)-methyl]-aminocarbonyl]-methyl]-phenyl]-propionsäure-methylester (Produkt aus Beispiel IV) in 50 ml Tetrahydrofuran/Dimethylformamid (3:2) gibt man unter Kühlung im Eis/Wasser-Bad 3,4 g Kalium-tert.butylat. Man rührt 60 Minuten bei Raumtemperatur, gießt die Reaktionslösung in 1N Salzsäure und extrahiert die wässrige Phase dreimal mit Essigsäure-ethylester. Die vereinigten organischen Phasen werden mit Wasser gewaschen, über Natriumsulfat getrocknet und das Lösungsmittel abgedampft. Das Rohprodukt wird mit Essigsäure-ethylester verrieben, der Niederschlag abgesaugt und getrocknet.
Ausbeute: 2,6 g (16 % der Theorie bezogen auf die beiden letzten Stufen),
Schmelzpunkt: 245-250 °C

Massenspektrum: $M^+$ = 362
$R_f$-Wert: 0,43 (Kieselgel; Methylenchlorid/Methanol = 9:1)

Beispiel VI

1-(4-Cyanophenyl)-3-[4-(2-methoxycarbonyl-ethyl)-phenyl]-4-morpholino-3-pyrrolin-2-on

Eine Lösung von 0,50 g 1-(4-Cyanophenyl)-4-hydroxy-3-[4-(2-methoxycarbonyl-ethyl)-phenyl]-3-pyrrolin-2-on in 10 ml Morpholin, 200 ml Xylol und 60 ml Eisessig wird 5 Stunden am Wasserabscheider zum Rückfluß erhitzt. Unter vermindertem Druck wird bei 150 °C das Lösungsmittel eingedampft und der Rückstand mit Methylenchlorid/Methanol (20:1) über Kieselgel chromatographiert.
Ausbeute: 0,36 g (60 % der Theorie),
Schmelzpunkt: 196-200 °C
Massenspektrum: $M^+$ = 431
$R_f$-Wert: 0,74 (Kieselgel; Methylenchlorid/Methanol = 9:1)

Beispiel VII

1-(4-Cyanophenyl)-4-methoxy-3-[4-(2-methoxycarbonyl-ethyl)-phenyl]-3-pyrrolin-2-on und
1-(4-Cyanophenyl)-3-[4-(2-methoxycarbonyl-ethyl)-phenyl]-3-methyl-pyrrolidin-2,4-dion

Zu einer Lösung von 1,40 g 1-(4-Cyanophenyl)-4-hydroxy-3-[4-(2-methoxycarbonyl-ethyl)-phenyl]-3-pyrrolin-2-on in 30 ml absolutem Dimethylformamid gibt man bei -15 °C 0,46 g Kalium-tert.butylat. Man läßt auf 0 °C erwärmen und tropft 0,3 ml Methyliodid zu. Nach 2 Stunden werden 0,35 ml Methyliodid und nach 4 Stunden weitere 0,5 ml Methyliodid zugetropft und der Umsatz mittels Dünnschicht-Chromatographie kontrolliert. Nach 6 Stunden wird die Reaktionslösung auf Eis/Wasser gegossen und die wäßrige Phase mit Essigester extrahiert. Die organische Phase wird mit Wasser gewaschen, über Natriumsulfat getrocknet und das Lösungsmittel unter vermindertem Druck eingedampft. Das Rohprodukt wird mit Essigester/Cyclohexan (1:1) über Kieselgel chromatographiert.
Ausbeute: 660 mg 1-(4-Cyanophenyl)-3-[4-(2-methoxycarbonylethyl)-phenyl]-3-methyl-pyrrolidin-2,4-dion (45 % der Theorie),
Schmelzpunkt: 108-111 °C
$R_f$-Wert: 0,57 (Kieselgel; Essigester/Cyclohexan = 1:1)
Ausbeute: 470 mg 1-(4-Cyanophenyl)-4-methoxy-3-[4-(2-methoxycarbonyl-ethyl)-phenyl]-3-pyrrolin-2-on (32 % der Theorie),
Schmelzpunkt: 163-168 °C
$R_f$-Wert: 0,28 (Kieselgel; Essigester/Cyclohexan = 1:1)

Beispiel 1

1-(4-Amidinophenyl)-4-methoxy-3-[4-(2-methoxycarbonyl-ethyl)-phenyl]-3-pyrrolin-2-on-hydrochlorid

0,75 g 1-(4-Cyanophenyl)-4-methoxy-3-[4-(2-methoxycarbonylethyl)-phenyl]-3-pyrrolin-2-on werden in 200 ml absolutem Methanol suspendiert. Unter Kühlung im Eis/Wasserbad leitet man eine Stunde lang trockenen Chlorwasserstoff ein. Die Reaktionslösung wird bei Raumtemperatur gerührt und der Umsatz mittels Dünnschicht-Chromatographie kontrolliert. Nach 4 Stunden wird die Reaktionslösung bei einer Badtemperatur von 35 °C unter vermindertem Druck eingedampft. Der Rückstand wird in 150 ml absolutem Methanol gelöst und die Lösung unter Rühren mit 5 g Ammoniumcarbonat versetzt. Man rührt 16 Stunden bei Raumtemperatur, saugt den Niederschlag ab und wäscht mit Wasser nach.
Ausbeute: 0,48 g (56 % der Theorie),
Massenspektrum: $(M+H)^+$ = 394
$R_f$-Wert: 0,57 (Kieselgel; Methylenchlorid/Methanol/konz. Ammoniak = 2:1:0,25)
Analog werden folgende Verbindungen erhalten:

(1) 1-(4-Amidino)-3-[4-(2-methoxycarbonyl-ethyl)-phenyl]-4-morpholino-3-pyrrolin-2-on-hydrochlorid
Massenspektrum: $M^+$ = 448
$R_f$-Wert: 0,14 (Kieselgel; Methylenchlorid/Methanol/konz. Ammoniak = 4:1:0,25)

(2) 1-(4-Amidinophenyl)-4-hydroxy-3-[4-(2-methoxycarbonylethyl)-phenyl]-3-pyrrolin-2-on-hydrochlorid
Massenspektrum: $(M+H)^+$ = 380

$R_f$-Wert: 0,58 (Kieselgel; Methylenchlorid/Methanol/konz. Ammoniak = 2:1:0,25)
(3) 1-(4-Amidinophenyl)-4-methoxy-3-[4-(2-methoxycarbonylethenyl)-phenyl]-3-pyrrolin-2-on-hydrochlorid
(4) 1-(4-Amidinophenyl)-3-[4-(2-methoxycarbonyl-ethyl)-phenyl]-4-pyrrolidino-3-pyrrolin-2-on-hydrochlorid
(5) 1-(4-Amidinophenyl)-4-methoxy-3-[4-(2-methoxycarbonylethyl)-phenyl]-5-methyl-3-pyrrolin-2-on-hydrochlorid
(6) 1-(4-Amidinophenyl)-4-methoxy-3-[4-(2-methoxycarbonylethyl)-phenyl]-5-phenyl-3-pyrrolin-2-on-hydrochlorid
(7) 1-(4-Amidinophenyl)-3-[4-(2-methoxycarbonyl-ethyl)-phenyl]-4-methylamino-3-pyrrolin-2-on-hydrochlorid
(8) 1-(4-Amidinophenyl)-4-methoxy-3-[4-[(methoxycarbonyl)-methyloxy]-phenyl]-3-pyrrolin-2-on-hydrochlorid
(9) 1-(4-Amidinophenyl)-4-N,N-dimethylamino-3-[4-(2-methoxycarbonyl-ethyl)-phenyl]-3-pyrrolin-2-on-hydrochlorid
(10) 1-(4-Amidinophenyl)-3-[4-[N,N-di-(methoxycarbonyl-methyl)-amino]-phenyl]-4-methoxy-3-pyrrolin-2-on-hydrochlorid
(11) 1-(4-Amidinophenyl)-3-[4-(2-methoxycarbonyl-ethyl)-phenyl]-4-(N-phenylamino)-3-pyrrolin-2-on-hydrochlorid
(12) 1-(4-Amidinophenyl)-3-[4-(2-methoxycarbonyl-ethyl)-phenyl]-3-pyrrolin-2-on-hydrochlorid
(13) 1-(4-Amidinophenyl)-3-[4-(2-methoxycarbonyl-ethyl)-phenyl]-4-phenyl-3-pyrrolin-2-on-hydrochlorid
(14) 1-(4-Amidinophenyl)-4-[4-(2-methoxycarbonyl-ethyl)-phenyl]-3-pyrrolin-2-on-hydrochlorid
(15) 1-(4-Amidinophenyl)-4-[4-(2-methoxycarbonyl-ethyl)-phenyl]-pyrrolidin-2-on-hydrochlorid
(16) 3-(4-Amidinophenyl)-4-methoxy-1-[4-(2-methoxycarbonylethyl)-phenyl]-3-pyrrolin-2-on-hydrochlorid
(17) 1-(4-Amidinophenyl)-3-[4-(2-methoxycarbonyl-ethyl)-phenyl]-pyrrolidin-hydrochlorid
(18) 1-(5-Amidinopyrid-2-yl)-3-[4-(2-methoxycarbonyl-ethyl)-phenyl]-pyrrolidin-2-on-hydrochlorid
(19) 3-(4-Amidinophenyl)-4-methoxy-1-[4-(2-methoxycarbonylethyl)-cyclohexyl]-3-pyrrolin-2-on-hydrochlorid
(20) 3-(4-Amidinophenyl)-4-isopropoxy-1-[4-(2-methoxycarbonyl-ethyl)-phenyl]-3-pyrrolin-2-on-hydrochlorid
(21) 1-(4-Amidino-2-methylphenyl)-4-methoxy-3-[4-(2-methoxycarbonyl-ethyl)-phenyl]-3-pyrrolin-2-on-hydrochlorid
(22) 1-(4-Amidino-2-fluorphenyl)-4-methoxy-3-[4-(2-methoxycarbonyl-ethyl)-phenyl]-3-pyrrolin-2-on-hydrochlorid
(23) 1-(4-Amidinophenyl)-4-methoxy-3-[2-(2-methoxycarbonylethyl)-pyrimid-5-yl]-3-pyrrolin-2-on-hydrochlorid
(24) 1-(4-Amidinophenyl)-3-[4-(2-methoxycarbonyl-ethyl)-phenyl]-3-(morpholinocarbonyl-methyl)-pyrrolidin-2,4-dion-hydrochlorid
(25) 1-(4-Amidinophenyl)-5,5-dimethyl-4-methoxy-3-[4-(2-methoxycarbonyl-ethyl)-phenyl]-3-pyrrolin-2-on-hydrochlorid
(26) 4-(4-Acylpiperazin-1-yl)-1-(4-amidinophenyl)-3-[4-(2-methoxycarbonyl-ethyl)-phenyl]-3-pyrrolin-2-on-hydrochlorid
(27) 1-(4-Amidinophenyl)-4-benzyloxy-3-[4-(2-methoxycarbonylethyl)-phenyl]-3-pyrrolin-2-on-hydrochlorid
(28) 1-(4-Amidinophenyl)-3-[4-(2-methoxycarbonyl-ethyl)-phenyl]-3-methyl-pyrrolidin-2,4-dion-hydrochlorid
Massenspektrum: $M^+$ = 393
$R_f$-Wert: 0,26 (Kieselgel; Methylenchlorid/Methanol/konz. Ammoniak = 4:1:0,25)
(29) 1-(4-Amidinophenyl)-3-[4-(2-methoxycarbonyl-ethyl)-phenyl]-4-piperidino-3-pyrrolin-2-on-hydrochlorid
(30) 3-(4-Amidinophenyl)-4-methoxy-1-[4-(2,2-dimethyl-2-methoxycarbonyl-ethyl)-phenyl]-3-pyrrolin-2-on-hydrochlorid
(31) 3-(4-Amidinophenyl)-4-ethoxy-1-[4-(2-methoxycarbonylethyl)-phenyl]-3-pyrrolin-2-on-hydrochlorid
(32) 1-(4-Amidinophenyl)-4-[4-(2-methoxycarbonyl-ethyl)-piperidino]-3-phenyl-3-pyrrolin-2-on-hydrochlorid
(33) 1-(4-Amidinophenyl)-4-methoxy-3-[3,4-di-[(methoxycarbonyl)-methyloxy]-phenyl]-3-pyrrolin-2-on-hydrochlorid
(34) 1-(4-Amidinophenyl)-3-[2-chlor-4-(2-methoxycarbonylethyl)-phenyl]-4-methoxy-3-pyrrolin-2-on-hydrochlorid
(35) 1-(4-Amidinophenyl)-3-[4-(2-methoxycarbonyl-ethyl)-phenyl]-4-thiomorpholino-3-pyrrolin-2-on-hydrochlorid
(36) 1-(4-Amidinophenyl)-4-methoxy-3-[4-[N-(methoxycarbonylmethyl)-amino]-phenyl]-3-pyrrolin-2-on-hydrochlorid

(37) 1-(4-Amidinophenyl)-4-methoxy-3-[4-[N-(methoxycarbonylmethyl)-methylamino]-phenyl]-3-pyrrolin-2-on-hydrochlorid

(38) 3-(4-Amidinophenyl)-4-methoxy-1-[1-(2-methoxycarbonylethyl)-piperid-4-yl]-3-pyrrolin-2-on-hydrochlorid

(39) 1-(4-Amidinophenyl)-4-methoxy-3-[4-(3-methoxycarbonylpropyl)-phenyl]-3-pyrrolin-2-on-hydrochlorid

(40) 4-Methoxy-3-[4-(2-methoxycarbonyl-ethyl)-phenyl]-1-[4-(N-methylamidino)-phenyl]-3-pyrrolin-2-on-hydrochlorid

Der Iminoester wird in absolutem Methanol gelöst und mit einem 20-fachen Überschuß einer methanolischen Methylaminlösung umgesetzt.

(41) 1-(4-Amidinophenyl)-4-methoxy-3-[4-[(methoxycarbonyl)-methylsulfonyl]-phenyl]-3-pyrrolin-2-on-hydrochlorid

(42) 1-(4-Amidinophenyl)-4-methoxy-3-[4-[(methoxycarbonyl)-methylsulfenyl]-phenyl]-3-pyrrolin-2-on-hydrochlorid

(43) 1-(4-Amidinophenyl)-4-(1,1-dioxido-thiomorpholino)-3-[4-(2-methoxycarbonyl-ethyl)-phenyl]-3-pyrrolin-2-on-hydrochlorid

(44) 1-(4-Amidinophenyl)-4-methoxy-3-[4-(2-phosphonoethyl)-phenyl]-3-pyrrolin-2-on-hydrochlorid

(45) 1-(4-Amidinophenyl)-4-methoxy-3-[4-[2-(0-methyl-phosphono)-ethyl]-phenyl]-3-pyrrolin-2-on-hydrochlorid

(46) 1-(4-Amidinophenyl)-4-methoxy-3-[4-[2-(tetrazol-5-yl)-ethyl]-phenyl]-3-pyrrolin-2-on-hydrochlorid

(47) 3-(4-Amidinophenyl)-1-[4-[2-amino-2-(methoxycarbonyl)-ethyl]-phenyl]-4-methoxy-3-pyrrolin-2-on-hydrochlorid

(48) 3-(4-Amidinophenyl)-1-[4-[2-hydroxy-2-(methoxycarbonyl)-ethyl]-phenyl]-4-methoxy-3-pyrrolin-2-on-hydrochlorid

(49) 1-(4-Amidinophenyl)-4-methoxy-3-[3-[(methoxycarbonyl)-methyloxy]-phenyl]-3-pyrrolin-2-on-hydrochlorid

(50) 1-(4-Amidinophenyl)-3-benzyl-3-[4-(2-methoxycarbonylethyl)-phenyl]-pyrrolidin-2,4-dion-hydrochlorid

(51) 1-(4-Amidinophenyl)-3-[4-(2-methoxycarbonyl-ethyl)-phenyl]-4-(morpholinocarbonyl-methoxy)-3-pyrrolin-2-on-hydrochlorid

(52) 1-[4-[2-(Acetylamino)-2-(methoxycarbonyl)-ethyl]-phenyl]-3-(4-amidinophenyl)-4-methoxy-3-pyrrolin-2-on-hydrochlorid

(53) 3-(4-Amidinophenyl)-4-methoxy-1-[4-[2-(methansulfonylamino)-2-(methoxycarbonyl)-ethyl]-phenyl]-3-pyrrolin-2-on-hydrochlorid

## Beispiel 2

### 1-(4-Amidinophenyl)-3-[4-(2-carboxy-ethyl)-phenyl]-4-methoxy-3-pyrrolin-2-on-hydrochlorid

Eine Lösung von 150 mg 1-(4-Amidinophenyl)-4-methoxy-3-[4-(2-methoxycarbonyl-ethyl)-phenyl]-3-pyrrolin-2-on und 64 mg Lithiumhydroxid-monohydrat in einem Gemisch aus 10 ml Tetrahydrofuran und 8 ml Wasser wird 2 Stunden bei Raumtemperatur gerührt. Anschließend wird mit 2 ml 1N Salzsäure angesäuert und das Tetrahydrofuran im Vakuum abgedampft. Der Niederschlag wird abgenutscht und mit Wasser gewaschen.

Ausbeute: 130 mg (90 % der Theorie),

Massenspektrum: $(M+H)^+$ = 380

$R_f$-Wert: 0,20 (Kieselgel; Methylenchlorid/Methanol/konz. Ammoniak = 2:1:0,25)

Analog werden folgende Verbindungen erhalten:

(1) 1-(4-Amidinophenyl)-3-[4-(2-carboxy-ethenyl)-phenyl]-4-methoxy-3-pyrrolin-2-on-hydrochlorid

(2) 1-(4-Amidinophenyl)-3-[4-(2-carboxy-ethyl)-phenyl]-4-hydroxy-3-pyrrolin-2-on-hydrochlorid

Massenspektrum: $(M+H)^+$ = 366

$R_f$-Wert: 0,16 (Kieselgel; Methylenchlorid/Methanol/konz. Ammoniak = 2:1:0,25)

(3) 1-(4-Amidinophenyl)-3-[4-(2-carboxy-ethyl)-phenyl]-4-morpholino-3-pyrrolin-2-on-hydrochlorid

Massenspektrum: $(M+H)^+$ = 435

$R_f$-Wert: 0,43 (Kieselgel; Methylenchlorid/Methanol/konz. Ammoniak = 2:1:0,25)

(4) 1-(4-Amidinophenyl)-3-[4-(2-carboxy-ethyl)-phenyl]-4-pyrrolidino-3-pyrrolin-2-on-hydrochlorid

(5) 1-(4-Amidinophenyl)-3-[4-(2-carboxy-ethyl)-phenyl]-4-methoxy-5-methyl-3-pyrrolin-2-on-hydrochlorid

(6) 1-(4-Amidinophenyl)-3-[4-(2-carboxy-ethyl)-phenyl]-4-methoxy-5-phenyl-3-pyrrolin-2-on-hydrochlorid

(7) 1-(4-Amidinophenyl)-3-[4-(2-carboxy-ethyl)-phenyl]-4-methylamino-3-pyrrolin-2-on-hydrochlorid

(8) 1-(4-Amidinophenyl)-3-[4-(carboxymethyloxy)-phenyl]-4-methoxy-3-pyrrolin-2-on-hydrochlorid

(9) 1-(4-Amidinophenyl)-3-[4-(2-carboxy-ethyl)-phenyl]-4-dimethylamino-3-pyrrolin-2-on-hydrochlorid
(10) 1-(4-Amidinophenyl)-3-[4-[N,N-di-(carboxymethyl)-amino]-phenyl]-4-methoxy-3-pyrrolin-2-on-hydrochlorid
(11) 1-(4-Amidinophenyl)-3-[4-(2-carboxy-ethyl)-phenyl]-4-(N-phenylamino)-3-pyrrolin-2-on-hydrochlorid
(12) 1-(4-Amidinophenyl)-3-[4-(2-carboxy-ethyl)-phenyl]-3-pyrrolin-2-on-hydrochlorid
(13) 1-(4-Amidinophenyl)-3-[4-(2-carboxy-ethyl)-phenyl]-pyrrolidin-2-on-hydrochlorid
Massenspektrum: $(M+H)^+ = 352$
$R_f$-Wert: 0,20 (Kieselgel; Methylenchlorid/Methanol/konz. Ammoniak = 2:1:0,25)
(14) 1-(4-Amidinophenyl)-4-[4-(2-carboxy-ethyl)-phenyl]-3-pyrrolin-2-on-hydrochlorid
(15) 1-(4-Amidinophenyl)-4-[4-(2-carboxy-ethyl)-phenyl]-pyrrolidin-2-on-hydrochlorid
(16) 3-(4-Amidinophenyl)-1-[4-(2-carboxy-ethyl)-phenyl]-4-methoxy-3-pyrrolin-2-on-hydrochlorid
(17) 1-(4-Amidinophenyl)-3-[4-(2-carboxy-ethyl)-phenyl]-pyrrolidin-hydrochlorid
(18) 1-(5-Amidinopyrid-2-yl)-3-[4-(2-carboxy-ethyl)-phenyl]-pyrrolidin-2-on-hydrochlorid
(19) 3-(4-Amidinophenyl)-1-[4-(2-carboxy-ethyl)-cyclohexyl]-4-methoxy-3-pyrrolin-2-on-hydrochlorid
(20) 3-(4-Amidinophenyl)-1-[4-(2-carboxy-ethyl)-phenyl]-4-isopropoxy-3-pyrrolin-2-on-hydrochlorid
(21) 1-(4-Amidinophenyl-2-methylphenyl)-3-[4-(2-carboxyethyl)-phenyl]-4-methoxy-3-pyrrolin-2-on-hydrochlorid
(22) 1-(4-Amidino-2-fluorphenyl)-3-[4-(2-carboxy-ethyl)-phenyl]-4-methoxy-3-pyrrolin-2-on-hydrochlorid
(23) 1-(4-Amidinophenyl)-3-[2-(2-carboxy-ethyl)-pyrimid5-yl]-4-methoxy-3-pyrrolin-2-on-hydrochlorid
(24) 1-(4-Amidinophenyl)-3-[4-(2-carboxy-ethyl)-phenyl]-4-phenyl-3-pyrrolin-2-on-hydrochlorid
(25) 1-(4-Amidinophenyl)-3-[4-(2-carboxy-ethyl)-phenyl]-5,5-dimethyl-4-methoxy-3-pyrrolin-2-on-hydrochlorid
(26) 4-(4-Acetylpiperazin-1-yl)-1-(4-amidinophenyl)-3-[4-(2-carboxy-ethyl)-phenyl]-3-pyrrolin-2-on-hydrochlorid
(27) 1-(4-Amidinophenyl)-4-benzyloxy-3-[4-(2-carboxy-ethyl)-phenyl]-3-pyrrolin-2-on-hydrochlorid
(28) 1-(4-Amidinophenyl)-3-[3-(carboxy-methyloxy)-phenyl]-4-methoxy-3-pyrrolin-2-on-hydrochlorid
(29) 1-(4-Amidinophenyl)-3-[4-(2-carboxy-ethyl)-phenyl]-4-piperidino-3-pyrrolin-2-on-hydrochlorid
(30) 3-(4-Amidinophenyl)-1-[4-(2-carboxy-2,2-dimethyl-ethyl)-phenyl]-4-methoxy-3-pyrrolin-2-on-hydrochlorid
(31) 3-(4-Amidinophenyl)-1-[4-(2-carboxy-ethyl)-phenyl]-4-ethoxy-3-pyrrolin-2-on-hydrochlorid
(32) 1-(4-Amidinophenyl)-4-[4-(2-carboxy-ethyl)-piperidino]-3-phenyl-3-pyrrolin-2-on-hydrochlorid
(33) 1-(4-Amidinophenyl)-3-[3,4-di-[(carboxymethyl)-oxy]-phenyl]-4-methoxy-3-pyrrolin-2-on-hydrochlorid
(34) 1-(4-Amidinophenyl)-3-[2-chlor-4-(2-carboxy-ethyl)-phenyl]-4-methoxy-3-pyrrolin-2-on-hydrochlorid
(35) 1-(4-Amidinophenyl)-3-[4-(2-carboxy-ethyl)-phenyl]-4-thiomorpholino-3-pyrrolin-2-on-hydrochlorid
(36) 1-(4-Amidinophenyl)-3-[4-(2-carboxy-ethyl)-phenyl]-4-(1-oxido-thiomorpholino)-3-pyrrolin-2-on-hydrochlorid
(37) 1-(4-Amidinophenyl)-3-[4-(2-carboxy-ethyl)-phenyl]-4-(1,1-dioxido-thiomorpholino)-3-pyrrolin-2-on-hydrochlorid
(38) 1-(4-Amidinophenyl)-3-[4-(carboxymethyl-sulfenyl)-phenyl]-4-methoxy-3-pyrrolin-2-on-hydrochlorid
(39) 1-(4-Amidinophenyl)-3-[4-(carboxymethyl-sulfinyl)-phenyl]-4-methoxy-3-pyrrolin-2-on-hydrochlorid
(40) 1-(4-Amidinophenyl)-3-[4-(carboxymethyl-sulfonyl)-Phenyl]-4-methoxy-3-pyrrolin-2-on-hydrochlorid
(41) 1-(4-Amidinophenyl)-3-[4-(carboxymethyl-amino)-phenyl]-4-methoxy-3-pyrrolin-2-on-hydrochlorid
(42) 1-(4-Amidinophenyl)-3-[4-(N-carboxymethyl-methylamino)-phenyl]-4-methoxy-3-pyrrolin-2-on-hydrochlorid
(43) 3-(4-Amidinophenyl)-1-[1-(2-carboxy-ethyl)-piperid-4-yl]-4-methoxy-3-pyrrolin-2-on-hydrochlorid
(44) 1-(4-Amidinophenyl)-3-[4-(3-carboxy-propyl)-phenyl]-4-methoxy-3-pyrrolin-2-on-hydrochlorid
(45) 3-[4-(2-Carboxy-ethyl)-phenyl]-4-methoxy-1-[4-(N-methylamidino)-phenyl]-3-pyrrolin-2-on-hydrochlorid
(46) 1-(1-Amidinopiperid-4-yl)-3-[4-(2-carboxy-ethyl)-phenyl]-4-methoxy-3-pyrrolin-2-on-hydrochlorid
(47) 3-(4-Amidinophenyl)-1-[4-(2-amino-2-carboxy-ethyl)-phenyl]-4-methoxy-3-pyrrolin-2-on-hydrochlorid
(48) 3-(4-Amidinophenyl)-1-[4-(2-carboxy-2-hydroxy-ethyl)-phenyl]-4-methoxy-3-pyrrolin-2-on-hydrochlorid
(49) 1-[4-(1-Aminoethyl)-phenyl]-3-[4-(2-carboxy-ethyl)-phenyl]-3-pyrrolidin-2-on-hydrochlorid
(50) 1-(4-Aminomethyl-cyclohexyl)-3-[4-(2-carboxy-ethyl)-phenyl]-4-methoxy-3-pyrrolin-2-on-hydrochlorid
(51) 1-[4-(1-Amino-1-methyl-ethyl)-phenyl]-3-[4-(2-carboxy-ethyl)-phenyl]-4-methoxy-3-pyrrolin-2-on-hydrochlorid
(52) 1-(1-Amino-1,2,3,4-tetrahydro-naphth-6-yl)-3-[4-(2-carboxy-ethyl)-phenyl]-4-methoxy-3-pyrrolin-2-on-hydrochlorid

(53) 1-(1-Amino-indan-5-yl)-3-[4-(2-carboxy-ethyl)-phenyl]-4-methoxy-3-pyrrolin-2-on-hydrochlorid
(54) 1-[3-(2-Aminoethyl)-phenyl]-3-[4-(2-carboxy-ethyl)-phenyl]-4-methoxy-3-pyrrolin-2-on-hydrochlorid
(55) 1-(4-Aminomethyl-phenyl)-3-[4-(2-carboxy-ethyl)-phenyl]-4-methoxy-3-pyrrolin-2-on-hydrochlorid
Massenspektrum: $(M+H)^+$ = 367
$R_f$-Wert: 0,24 (Kieselgel; Methylenchlorid/Methanol/konz. Ammoniak = 4:1:0,25)
(56) 1-[4-(Allyloxycarbonyl-amidino)-phenyl]-3-[4-(2-carboxy-ethyl)-phenyl]-4-methoxy-3-pyrrolin-2-on-hydrochlorid
(57) 1-[4-(Allyloxycarbonyl-amidino)-phenyl]-3-[4-(2-carboxy-ethyl)-phenyl]-4-morpholino-3-pyrrolin-2-on-hydrochlorid
(58) 1-(4-Aminomethyl-phenyl)-3-[4-(2-carboxy-ethyl)-phenyl]-pyrrolidin-2-on-hydrochlorid
Massenspektrum: $M^+$ = 338
$R_f$-Wert: 0,32 (Kieselgel; Methylenchlorid/Methanol/konz. Ammoniak = 4:1:0,25)
(59) 3-[4-(3-Carboxy-propyl)-phenyl]-4-methoxy-1-piperid-4-yl-3-pyrrolin-2-on-hydrochlorid
(60) 1-(4-Amidinophenyl)-3-[4-(2-carboxy-ethyl)-phenyl]-4-(morpholinocarbonyl-methoxy)-3-pyrrolin-2-on-hydrochlorid
(61) 1-[4-(2-Acetylamino-2-carboxy-ethyl)-phenyl]-3-(4-amidinophenyl)-4-methoxy-3-pyrrolin-2-on-hydrochlorid
(62) 3-(4-Amidinophenyl-1-[4-[2-carboxy-2-(methansulfonyl-amino)-ethyl]-phenyl]-4-methoxy-3-pyrrolin-2-on-hydrochlorid

## Beispiel 3

1-(1-Amidinopiperid-4-yl)-4-methoxy-3-[4-(2-methoxycarbonyl-ethyl)-phenyl]-3-pyrrolin-2-on

Herstellung aus 4-Methoxy-3-[4-(2-methoxycarbonyl-ethyl)-phenyl]-1-(piperid-4-yl)-3-pyrrolin-2-on und S-Ethylisothioharnstoff-hydrobromid durch vierstündiges Erwärmen auf 100°C in Dimethylformamid in Gegenwart von Natriumcarbonat.

## Beispiel 4

1-[4-(1-Aminoethyl)-phenyl]-3-[4-(2-methoxycarbonyl-ethyl)-phenyl]-pyrrolidin-2-on

Herstellung aus 1-[4-[1-[(tert.Butyloxycarbonyl)-amino]-ethyl]-phenyl]-3-[4-(2-methoxycarbonyl-ethyl)-phenyl]-pyrrolidin-2-on durch zweistündiges Rühren in einem 1:1 Gemisch aus Methylenchlorid und Trifluoressigsäure.

Analog werden folgende Verbindungen erhalten:
(1) 1-[4-Aminomethyl-cyclohexyl]-4-methoxy-3-[4-(2-methoxy-carbonyl-ethyl)-phenyl]-3-pyrrolin-2-on
(2) 1-[4-(1-Amino-1-methyl-ethyl)-phenyl]-4-methoxy-3-[4-(2-methoxycarbonyl-ethyl)-phenyl]-3-pyrrolin-2-on
(3) 1-[1-Amino-1,2,3,4-tetrahydro-naphth-6-yl]-4-methoxy-3-[4-(2-methoxycarbonyl-ethyl)-phenyl]-3-pyrrolin-2-on
(4) 1-[1-Amino-indan-5-yl)-4-methoxy-3-[4-(2-methoxycarbonyl-ethyl)-phenyl]-3-pyrrolin-2-on
(5) 1-[3-(2-Aminoethyl)-phenyl]-4-methoxy-3-[4-(2-methoxycarbonyl-ethyl)-phenyl]-3-pyrrolin-2-on
(6) 4-Methoxy-3-[4-(3-methoxycarbonyl-propyl)-phenyl]-1-piperid-4-yl-3-pyrrolin-2-on

## Beispiel 5

1-(4-Aminomethyl-phenyl)-3-[4-(2-methoxycarbonyl-ethyl)-phenyl]-pyrrolidin-2-on und
1-(4-Aminomethyl-phenyl)-4-methoxy-3-[4-(2-methoxycarbonyl-ethyl)-phenyl]-3-pyrrolin-2-on

Eine Lösung von 0,45 g 1-(4-Cyanophenyl)-4-methoxy-3-[4-(2-methoxycarbonyl-ethyl)-phenyl]-4-pyrrolin-2-on in 100 ml absolutem Methanol und 100 ml mit Chlorwasserstoff gesättigtem absolutem Methanol wird in Gegenwart von 0,2 g 10 % Palladium auf Aktivkohle und einem Wasserstoff-Druck von 5 bar bei Raumtemperatur 7 Stunden hydriert. Anschließend wird der Katalysator abfiltriert und das Filtrat unter vermindertem Druck eingedampft. Der Rückstand wird in Wasser gelöst und mit 1N Natronlauge versetzt. Die wässrige Phase wird zweimal mit Essigsäure-ethylester und einmal mit Methylenchlorid extrahiert. Die vereinigten organischen Phasen werden getrocknet und das Lösungsmittel eingedampft. Der verbleibende Rückstand wird mit Methylenchlorid/Methanol/konz. Ammoniak (10:1:0,1) über Kieselgel chromatographiert.
Ausbeute: 100 mg 1-(4-Aminomethyl-phenyl)-3-[4-(2-methoxy-carbonyl-ethyl)-phenyl]-pyrrolidin-2-on (24 % der Theorie) (= Verbindung A),
Massenspektrum: $M^+$ = 352
$R_f$-Wert: 0,33 (Kieselgel; Methylenchlorid/Methanol/konz. Ammoniak = 10:1:0,1)
Ausbeute: 140 mg 1-(4-Aminomethyl-phenyl)-4-methoxy-3-[4-(2-methoxycarbonyl-ethyl)-phenyl]-3-pyrrolin-2-on-hydrochlorid (31 % der Theorie) (= Verbindung B),
Massenspektrum: $M^+$ = 380
$R_f$-Wert: 0,28 (Kieselgel; Methylenchlorid/Methanol/konz. Ammoniak = 10:1:0,1)

Beispiel 6

4-Methoxy-1-[4-(methoxycarbonyl-amidino)-phenyl]-3-[4-(2-methoxycarbonyl-ethyl)-phenyl]-3-pyrrolin-2-on

Hergestellt aus 1-(4-Amidinophenyl)-4-methoxy-3-[4-(2-methoxycarbonyl-ethyl)-phenyl]-3-pyrrolin-2-on und Chlorameisensäuremethylester in Methylenchlorid und Zugabe von 0,1N Natronlauge unter kräftigem Rühren.

Analog werden folgende Verbindungen erhalten:

(1) 4-Methoxy-1-[4-(methoxycarbonyl-amidino)-phenyl]-3-[4-[2-(isopropoxycarbonyl)-ethyl]-phenyl]-3-pyrrolin-2-on

(2) 1-[4-[[(1-Acetyloxy-ethyl)-oxycarbonyl]-amidino]-phenyl]-4-methoxy-3-[4-(2-methoxycarbonyl-ethyl)-phenyl]-3-pyrrolin-2-on
Es wird Kohlensäure-(1-acetyloxy-ethyl)-(4-nitrophenyl)-ester und N-Ethyl-diisopropylamin eingesetzt.

(3) 1-[4-[[(Acetyloxymethyl)-oxycarbonyl]-amidino]-phenyl]-4-methoxy-3-[4-(2-methoxycarbonyl-ethyl)-phenyl]-3-pyrrolin-2-on
Es wird Kohlensäure-acetyloxymethyl-(4-nitrophenyl)-ester und N-Ethyl-diisopropylamin eingesetzt.

(4) 1-[4-(Ethoxycarbonyl-amidino)-phenyl]-4-methoxy-3-[4-(2-methoxycarbonyl-ethyl)-phenyl]-3-pyrrolin-2-on
Es wird Chlorameisensäure-ethylester eingesetzt.

(5) 1-[4-(Allyloxycarbonyl-amidino)-phenyl]-4-methoxy-3-[4-(2-methoxycarbonyl-ethyl)-phenyl]-3-pyrrolin-2-on
Es wird Chlorameisensäure-allylester eingesetzt.

(6) 1-[4-(Allyloxycarbonyl-amidino)-phenyl]-3-[4-(2-methoxy-carbonyl-ethyl)-phenyl]-4-morpholino-3-pyrrolin-2-on
Es wird Chlorameisensäure-allylester eingesetzt.

(7) 1-[4-(Methoxycarbonyl-amidino)-phenyl]-3-[4-(2-methoxy-carbonyl-ethyl)-phenyl]-4-morpholino-3-pyrrolin-2-on

(8) 3-[4-(2-Ethoxycarbonyl-ethyl)-phenyl]-4-methoxy-1-[4-(methoxycarbonyl-amidino)-phenyl]-3-pyrrolin-2-on

Beispiel 7

1-(4-Amidinophenyl)-3-[4-(2-methoxycarbonyl-ethyl)-phenyl]-4-(1-oxido-thiomorpholino)-3-pyrrolin-2-on

Herstellung durch Zutropfen einer Lösung von Jodbenzoldichlorid (1 Äquivalent) in wässrigem Pyridin zu einer gerührten Lösung von 1-(4-Amidinophenyl)-3-[4-(2-methoxy-carbonyl-ethyl)-phenyl]-4-thiomorpholino-3-pyrrolin-2-on (1 Äquivalent) in 20%igem wässrigem Pyridin bei 20 °C unter Ausschluß von direktem Sonnenlicht.

Analog wird folgende Verbindung erhalten:

(1) 1-(4-Amidinophenyl)-4-methoxy-3-[4-(methoxycarbonyl-methylsulfinyl)-phenyl]-3-pyrrolin-2-on

Beispiel 8

1-(4-Amidinophenyl)-3-[4-(2-methoxycarbonyl-ethyl)-phenyl]-pyrrolidin-2-on

Eine Lösung von 270 mg 1-(4-Amidinophenyl)-3-[4-(2-carboxy-ethyl)-phenyl]-4-methoxy-3-pyrrolin-2-on-hydrochlorid in 180 ml absolutem Methanol und 10 ml mit Chlorwasserstoff gesättigtem absolutem Methanol wird in Gegenwart von 100 mg 10 % Palladium auf Aktivkohle und einem Wasserstoff-Druck von 5

bar bei Raumtemperatur 2,5 Stunden hydriert. Man gibt weitere 100 mg 10 % Palladium auf Aktivkohle zu und hydriert weitere 4 Stunden. Anschließend wird der Katalysator abfiltriert und das Filtrat unter vermindertem Druck eingedampft. Der Rückstand wird in Wasser gelöst und mit 1N Natronlauge versetzt. Die wässrige Phase wird dreimal mit Methylenchlorid extrahiert, die organische Phase getrocknet und das Lösungsmittel eingedampft. Der verbleibende Rückstand wird mit Methylenchlorid/Methanol/konz. Ammoniak (4:1:0,25) über Kieselgel chromatographiert.
Ausbeute: 120 mg (49 % der Theorie),
Massenspektrum: $(M+H)^+ = 366$
$R_f$-Wert: 0,29 (Kieselgel; Methylenchlorid/Methanol/konz. Ammoniak = 2:1:0,25)

Beispiel 9

1-(4-Amidinophenyl)-3-[4-[2-(cyclohexyloxycarbonyl)-ethyl]-phenyl]-4-methoxy-3-pyrrolin-2-on-hydrochlorid

Herstellung durch einstündiges Durchleiten von trockenem Chlorwasserstoff durch eine Lösung von 1-(4-Amidinophenyl)-3-[4-(2-carboxy-ethyl)-phenyl]-4-methoxy-3-pyrrolin-2-on und einem Überschuß von Cyclohexanol in Methylenchlorid und anschließendem Erwärmen auf 40 ° C für eine Stunde.

Analog werden folgende Verbindungen erhalten:

(1) 1-(4-Amidinophenyl)-3-[4-[2-[(2-morpholino-ethyl)-oxycarbonyl]-ethyl]-phenyl]-3-pyrrolin-2-on-hydrochlorid
Es wird 2-Morpholino-ethanol eingesetzt

(2) 1-(4-Amidinophenyl)-3-[4-[2-(isopropoxycarbonyl)-ethyl]-phenyl]-4-methoxy-3-pyrrolin-2-on-hydrochlorid
Die Umsetzung wird in Isopropanol durchgeführt.

(3) 1-(4-Amidinophenyl)-3-[4-[2-(benzyloxycarbonyl)-ethyl]-phenyl]-4-methoxy-3-pyrrolin-2-on-hydrochlorid
Die Umsetzung wird in Benzylalkohol durchgeführt.

(4) 1-(4-Amidinophenyl)-3-[4-[2-(ethoxycarbonyl)-ethyl]-phenyl]-4-methoxy-3-pyrrolin-2-on-hydrochlorid
Die Umsetzung wird in Ethanol durchgeführt.

Beispiel 10

1-[4-(Allyloxycarbonyl-amidino)-phenyl]-4-methoxy-3-[4-[2-[(pivaloyloxymethyl)-oxycarbonyl]-ethyl]-phenyl]-3-pyrrolin-2-on

Herstellung durch dreitägiges Rühren einer Suspension von 1-[4-(Allyloxycarbonyl-amidino)-phenyl]-3-[4-(2-carboxy-ethyl)-phenyl]-4-methoxy-3-pyrrolin-2-on, 2 Äquivalenten Pivalinsäure-chlormethylester, 2 Äquivalenten Kaliumiodid, 2 Äquivalenten Kaliumhydrogencarbonat und 2 Äquivalenten Kaliumcarbonat in Dimethylformamid bei Raumtemperatur.

Analog werden folgende Verbindungen erhalten:

(1) 1-[4-(Allyloxycarbonyl-amidino)-phenyl]-3-[4-[2-[[1-[(ethoxycarbonyl)-oxy]-ethyl]-oxycarbonyl]-ethyl]-phenyl]-4-methoxy-3-pyrrolin-2-on
Die Umsetzung wird mit 1-(Ethoxycarbonyloxy)-ethylchlorid in Dimethylsulfoxid durchgeführt.

(2) 1-[4-(Allyloxycarbonyl-amidino)-phenyl]-3-[4-[2-[[1-[(cyclohexyloxycarbonyl)-oxy]-ethyl]-oxycarbonyl]-ethyl]-phenyl]-4-morpholino-3-pyrrolin-2-on
Die Umsetzung wird mit 1-(Cyclohexyloxycarbonyloxy)-ethylchlorid in Dimethylsulfoxid durchgeführt.

(3) 1-[4-(Allyloxycarbonyl-amidino)-phenyl]-4-morpholino-3-[4-[2-[(pivaloyloxymethyl)-oxycarbonyl]-ethyl]-phenyl]-3-pyrrolin-2-on

Beispiel 11

1-[4-(Allyloxycarbonyl-amidino)-phenyl]-3-[4-[2-[[(dimethylaminocarbonyl)-methyl]-oxycarbonyl]-ethyl]-phenyl]-4-methoxy-3-pyrrolin-2-on

Herstellung durch 16-stündiges Rühren einer Suspension von 1-[4-(Allyloxycarbonyl-amidino)-phenyl]-3-[4-[2-carboxy-ethyl-phenyl]-4-methoxy-3-pyrrolin-2-on, 1 Äquivalent 2-Chlor-N,N-dimethylacetamid, 1 Äquivalent Triethylamin und 0,1 Äquivalent Natriumiodid in absolutem Dimethylformamid bei Raumtemperatur.

Analog werden folgende Verbindungen erhalten:

(1) 1-[4-(Allyloxycarbonyl-amidino)-phenyl]-4-methoxy-3-[4-[2-[[(morpholinocarbonyl)-methyl]-oxycarbonyl]-ethyl]-phenyl]-3-pyrrolin-2-on
Es wird 1-(2-Chloracetyl)-morpholin eingesetzt.
(2) 1-[4-(Allyloxycarbonyl-amidino)-phenyl]-3-[4-[2-[[(dimethylaminocarbonyl)-methyl]-oxycarbonyl]-ethyl]-phenyl]-4-morpholino-3-pyrrolin-2-on
(3) 1-[4-(Allyloxycarbonyl-amidino)-phenyl]-4-morpholino-3-[4-[2-[[(morpholinocarbonyl)-methyl]-oxycarbonyl]-ethyl]-phenyl]-3-pyrrolin-2-on
Es wird 1-(2-Chloracetyl)-morpholin eingesetzt.

Beispiel 12

1-(4-Amidinophenyl)-4-methoxy-3-[4-[2-[(pivaloyloxymethyl)-oxycarbonyl]-ethyl]-phenyl]-3-pyrrolin-2-on

Herstellung durch Zutropfen eines Äquivalents Morpholin zu 1-[4-(Allyloxycarbonyl-amidino)-phenyl]-4-methoxy-3-[4-[2-[(pivaloyloxymethyl)-oxycarbonyl]-ethyl]-phenyl]-3-pyrrolin-2-on und 0,1 Äquivalenten Tetrakis-(triphenyl-phosphin)-palladium(O) in Tetrahydrofuran unter Inertgas und anschließendem einstündigem Rühren bei Raumtemperatur.

Analog werden folgende Verbindungen erhalten:
(1) 1-[4-(Amidinophenyl)-3-[4-[2-[[1-[(ethoxycarbonyl)-oxy]-ethyl]-oxycarbonyl]-ethyl]-phenyl]-4-methoxy-3-pyrrolin-2-on
(2) 1-[4-(Amidinophenyl)-3-[4-[2-[[(dimethylaminocarbonyl)-methyl]-oxycarbonyl]-ethyl]-phenyl]-4-methoxy-3-pyrrolin-2-on
(3) 1-[4-(Amidinophenyl)-4-methoxy-3-[4-[2-[[(morpholinocarbonyl)-methyl]-oxycarbonyl]-ethyl]-phenyl]-3-pyrrolin-2-on
(4) 1-[4-(Amidinophenyl)-3-[4-[2-[[(dimethylaminocarbonyl)-methyl]-oxycarbonyl]-ethyl]-phenyl]-4-morpholino-3-pyrrolin-2-on
(5) 1-[4-(Amidinophenyl)-4-morpholino-3-[4-[2-[[(morpholino-carbonyl)-methyl]-oxycarbonyl]-ethyl]-phenyl]-3-pyrrolin-2-on
(6) 1-[4-(Amidinophenyl)-3-[4-[2-[[1-[(cyclohexyloxycarbonyl)-oxy]-ethyl]-oxycarbonyl]-ethyl]-phenyl]-4-morpholino-3-pyrrolin-2-on
(7) 1-[4-(Amidinophenyl)-4-morpholino-3-[4-[2-[(pivaloyloxy-methyl)-oxycarbonyl]-ethyl]-phenyl]-3-pyrrolin-2-on

Beispiel 13

1-(4-Amidinophenyl)-3-[4-(2-carboxy-ethyl)-phenyl]-3-methyl-pyrrolidin-2,4-dion-hydrochlorid

Eine Lösung von 190 mg 1-(4-Amidinophenyl)-3-[4-(2-methoxy-carbonyl-ethyl)-phenyl]-3-methyl-pyrrolidin-2,4-dion in 200 ml 6N Salzsäure wird 4 Stunden bei Raumtemperatur gerührt. Der Niederschlag wird abgenutscht, mit wenig Wasser gewaschen und getrocknet (Ausbeute: 60 mg). Das Filtrat wird eingedampft, der kristalline Rückstand mit Wasser verrieben, abgenutscht und getrocknet (Ausbeute: 60 mg).
Gesamtausbeute: 120 mg (65 % der Theorie),
Massenspektrum: $(M+H)^+ = 380$
Schmelzpunkt: 275-280 °C
$R_f$-Wert: 0,14 (Kieselgel; Methylenchlorid/Methanol/konz. Ammoniak = 2:1:0,25)
Analog werden folgende Verbindungen erhalten:
(1) 1-(4-Amidinophenyl)-3-[4-(2-carboxy-ethyl)-phenyl]-3-(morpholinocarbonyl-methyl)-pyrrolidin-2,4-dion-hydrochlorid
(2) 1-(4-Amidinophenyl)-3-benzyl-3-[4-(2-carboxy-ethyl)phenyl]-pyrrolidin-2,4-dion-hydrochlorid

Beispiel 14

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke | ad 1,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 15

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 16

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 17

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 18

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 19

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

1. Cyclische Iminoderivate der allgemeinen Formel in der

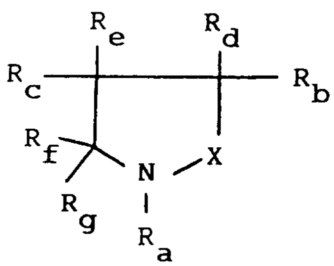

,(I)

einer der Reste $R_a$ oder $R_b$ eine A-B-Gruppe und
der andere der Reste $R_a$ oder $R_b$ eine F-E-D-Gruppe, in denen

A eine Aminogruppe, eine Aminoalkylgruppe mit 1 bis 4 Kohlenstoffatomen im Alkylteil, eine Amidino- oder Guanidinogruppe, wobei in den vorstehend erwähnten Amino-, Aminoalkyl-, Amidino- oder Guanidinogruppen eine Amino- oder Iminogruppe durch eine Alkylgruppe mit 1 bis 4 Kohlenstoffatomen oder durch eine in vivo abspaltbare Gruppe substituiert und eine Aminogruppe zusätzlich durch eine weitere Alkylgruppe mit 1 bis 4 Kohlenstoffatomen oder durch eine Aralkylgruppe substituiert sein kann,

B eine Phenylengruppe, die durch ein Fluor-, Chlor- oder Bromatom, durch eine oder zwei Alkylgruppen, durch eine Trifluormethyl-, Hydroxy-, Alkoxy-, Alkylsulfenyl-, Alkylsulfinyl-, Alkylsulfonyl-, Nitro-, $(R_1)_2N$-, N-Alkylcarbonyl-$NR_1$- oder N-Alkansulfonyl-$NR_1$-Gruppe, wobei $R_1$ ein Wasserstoffatom, eine Alkyl- oder Aralkylgruppe darstellt, substituiert sein kann,

eine Pyridinylen-, Pyrimidinylen-, Pyrazinylen-, Pyridazinylen- oder Triazinylengruppe, die jeweils im Kohlenstoffgerüst durch eine Alkylgruppe substituiert und in denen zusätzlich eine oder zwei -CH=N-Gruppen jeweils durch eine -CO-$NR_1$-Gruppe ersetzt sein können, wobei $R_1$ wie vorstehend erwähnt definiert ist und zwischen dem Stickstoffatom der -CO-$NR_1$-Gruppe und dem Stickstoffatom des Restes A oder des cyclischen Iminorestes, an den der Rest B geknüpft ist, mindestens 2 Kohlenstoffatome liegen müssen,

eine 1,3-Pyrrolidinylen-, 1,3-Piperidinylen-, 1,4-Piperidinylen-, 1,3-Hexahydroazepinylen-, 1,4-Hexahydroazepinylen-, 1,4-Piperazinylen- oder 1,4-Homopiperazinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf, und

eine Cycloalkylengruppe mit 5 bis 7 Kohlenstoffatomen, in der eine Methylengruppe durch eine -$NR_1$-Gruppe, in der $R_1$ wie vorstehend erwähnt definiert ist, ersetzt sein kann, wobei zusätzlich zwischen dem Stickstoffatom der -$NR_1$-Gruppe und einem Stickstoffatom des Restes A oder des cyclischen Iminorestes mindestens zwei Kohlenstoffatome liegen müssen, und

eine Indanyliden-, 1,2,3,4-Tetrahydronaphthyliden- oder 5H-Benzocycloheptenylidengruppe oder

A und B zusammen eine gegebenenfalls in 1-Stellung durch den Rest $R_1$ oder durch eine in vivo abspaltbare Gruppe substituierte Pyrrolidinyl-, Piperidinyl-, Hexahydroazepinyl- oder Piperazinogruppe, wobei $R_1$ wie vorstehend erwähnt definiert ist,

D eine Phenylengruppe, die durch ein Fluor-, Chlor- oder Bromatom, durch eine oder zwei Alkylgruppen, durch eine Trifluormethyl-, Hydroxy-, Alkoxy-, Alkylsulfenyl-, Alkylsulfinyl-, Alkylsulfonyl-, Nitro-, $(R_1)_2N$-, N-Alkylcarbonyl-$NR_1$-, N-Alkansulfonyl-$NR_1$-, Carboxymethoxy- oder Alkoxycarbonylmethoxygruppe substituiert sein kann, wobei $R_1$ wie vorstehend erwähnt definiert ist,

eine Cycloalkylengruppe mit 5 bis 7 Kohlenstoffatomen,

eine Pyridinylen-, Pyrimidinylen-, Pyrazinylen-, Pyridazinylen- oder Triazinylengruppe, die jeweils im Kohlenstoffgerüst durch eine Alkylgruppe substituiert sein kann, wobei zusätzlich eine -CH=N-Gruppe durch eine $-CO-NR_2$-Gruppe ersetzt sein kann, wobei $R_2$ ein Wasserstoffatom, eine Alkyl- oder Aralkylgruppe oder auch eine Bindung mit dem Rest E darstellt, wenn sich immer zwischen einem Heteroatom des Restes E oder dem Stickstoffatom des cyclischen Iminorestes mit dem der Rest D verknüpft ist und dem Stickstoffatom der $-CONR_2$-Gruppe mindestens zwei Kohlenstoffatome befinden,

eine 1,3-Pyrrolidinylen-, 1,3-Piperidinylen-, 1,4-Piperidinylen-, 1,3-Hexahydroazepinylen- oder 1,4-Hexahydroazepinylen-, 1,4-Piperazinylen- oder 1,4-Homopiperazinylengruppe, welche über die Position 1 mit dem Rest E oder mit dem cyclischen Iminorest verknüpft sein können und in denen eine zu dem Stickstoffatom benachbarte Methylengruppe durch eine Carbonylgruppe ersetzt sein kann, wobei jedoch die Verknüpfung nicht über eine Stickstoff-Stickstoff-Bindung erfolgen darf,

eine Cycloalkylengruppe mit 5 bis 7 Kohlenstoffatomen, in der eine Methylengruppe durch eine $-NR_1$-Gruppe ersetzt ist, wobei $R_1$ wie vorstehend erwähnt definiert ist,

E eine gegebenenfalls durch eine Hydroxy-, Alkoxy-, $(R_1)_2N$-, Alkyl-CO-$NR_1$-, Aralkyl-CO-$NR_1$-, Aryl-CO-$NR_1$-, $C_{1-5}$-Alkyl-$SO_2$-$NR_1$-, Arylalkyl-$SO_2$-$NR_1$-, Aryl-$SO_2$-$NR_1$-, $(R_1)_2N$-CO-$NR_1$-, Alkoxy-CO-$NR_1$- oder Aralkoxy-CO-$NR_1$-Gruppe substituierte geradkettige oder verzweigte Alkylengruppe mit 1 bis 5 Kohlenstoffatomen, in denen die Reste $R_1$, die gleich oder verschieden sein können, wie eingangs erwähnt definiert sind, eine geradkettige oder verzweigte Alkenylengruppe mit 2 bis 5 Kohlenstoffatomen, wobei in einer Alkylengruppe mit 2 bis 4 Kohlenstoffatomen die mit dem Rest D verknüpfte Methylengruppe durch ein Sauerstoff- oder Schwefelatom, durch eine Sulfinyl-, Sulfonyl-, Imino-, Alkylimino-, Aralkylimino-, Alkylcarbonylimino-, Aralkylcarbonylimino-, Arylcarbonylimino-, Alkansulfonylimino-, Arylalkansulfonylimino-, Arylsulfonylimino-, Carboxymethylimino-, Alkoxycarbonylmethylimino- oder Aralkoxycarbonylmethyliminogruppe ersetzt sein kann, und

F eine in vivo zu einer Carboxylgruppe metabolisierbare Gruppe, eine Carboxylgruppe, eine Sulfo-, Phosphono-, O-Alkylphosphono- oder Tetrazol-5-ylgruppe darstellen, und entweder

(a) X eine Methylen- oder Carbonylgruppe,

$R_d$ und $R_e$ zusammen eine weitere Kohlenstoff-Kohlenstoff-Bindung,

$R_c$ ein Wasserstoffatom, eine Alkylgruppe mit 1 bis 5 Kohlenstoffatomen, eine Arylgruppe, eine Hydroxygruppe, eine geradkettige oder verzweigte Alkoxygruppe mit 1 bis 5 Kohlenstoffatomen, wobei eine Alkoxygruppe mit 1 bis 3 Kohlenstoffatomen in 1-, 2- oder 3-Stellung durch eine Carboxy-, Alkoxycarbonyl-, Aminocarbonyl-, Alkylaminocarbonyl-, Dialkylaminocarbonyl-, Cycloalkylaminocarbonyl-, Cycloalkylalkylaminocarbonyl-, Piperidinocarbonyl-, Morpholinocarbonyl-, Thiomorpholinocarbonyl-, 1-Oxidothiomorpholinocarbonyl- oder 1,1-Dioxidothiomorpholinocarbonylgruppe substituiert sein kann, eine Aralkoxy-, Alkylamino-, Dialkylamino-, Aralkylamino-, N-Alkyl-aralkylamino-, Arylamino-, N-Alkyl-arylamino-, Pyrrolidino-, Piperidino- oder Hexamethyleniminogruppe, wobei die Methylengruppe in 4-Stellung einer Piperidinogruppe durch ein Sauerstoff- oder Schwefelatom, durch eine Sulfinyl-, Sulfonyl-, Imino-, Alkylimino-, Aralkylimino-, Alkylcarbonylimino-, Aralkylcarbonylimino-, Arylcarbonylimino-, Alkansulfonylimino-, Arylalkansulfonylimino- oder Arylsulfonyliminogruppe ersetzt sein kann,

$R_f$ ein Wasserstoffatom, eine geradkettige oder verzweigte Alkylgruppe mit 1 bis 5 Kohlenstoffatomen, eine Aralkyl- oder Arylgruppe und

$R_g$ ein Wasserstoffatom oder eine Alkylgruppe oder

(b) X eine Carbonylgruppe,

$R_c$ und $R_e$ zusammen mit dem dazwischen liegenden Kohlenstoffatom eine Carbonylgruppe,

$R_d$ eine gegebenenfalls durch eine Aryl-, Carboxy-, Alkoxycarbonyl-, Aminocarbonyl-, Alkylaminocarbonyl-, Dialkylaminocarbonyl-, Cycloalkylaminocarbonyl-, Cycloalkylalkylaminocarbonyl-, Piperidinocarbonyl-, Morpholinocarbonyl-, Thiomorpholinocarbonyl-, 1-Oxidothiomorpholinocarbonyl- oder 1,1-Dioxidothiomorpholinocarbonylgruppe substituierte Alkylgruppe,

$R_f$ ein Wasserstoffatom, eine Alkyl- oder Arylgruppe und

$R_g$ ein Wasserstoffatom oder eine Alkylgruppe oder

(c) X eine Methylengruppe,

$R_f$ und $R_g$ zusammen mit dem dazwischen liegenden Kohlenstoffatom eine Carbonylgruppe,

$R_c$ ein Wasserstoffatom oder auch, wenn B oder D eine 1,3-Pyrrolidinylen-, 1,3-Piperidinylen-, 1,4-Piperidinylen-, 1,3-Hexahydroazepinylen-, 1,4-Hexahydroazepinylen-, 1,4-Piperazinylen- oder 1,4-Homopiperazinylengruppe, welche über die Position 1 mit dem cyclischen Iminorest verknüpft sind, darstellt, eine Arylgruppe und

$R_d$ und $R_e$ jeweils ein Wasserstoffatom oder zusammen eine weitere Kohlenstoff-Kohlenstoff-Bindung

oder
(d) X eine Methylen- oder Carbonylgruppe und
$R_c$ bis $R_f$ jeweils ein Wasserstoffatom bedeuten,
wobei unter "eine in vivo zu einer Carboxylgruppe metabolisierbare Gruppe" und unter "eine in vivo abspaltbare Gruppe" beispielsweise eine Estergruppe der Formeln

- CO - OR',
- CO - O - (HCR'') - O - CO - R''' und
- CO - O - (HCR'') - O - CO - OR''',

in denen
R' eine geradkettige oder verzweigte Alkylgruppe mit 1 bis 6 Kohlenstoffatomen, wobei die Methylgruppe durch eine $(R_1)_2$NCO-Gruppe, in der $R_1$ wie eingangs definiert ist, durch eine Pyridyl-, Cycloalkylaminocarbonyl-, Cycloalkylalkylaminocarbonyl-, Pyrrolidinocarbonyl-, Piperidinocarbonyl-, Morpholinocarbonyl-, Thiomorpholinocarbonyl-, 1-Oxidothiomorpholinocarbonyl- oder 1,1-Dioxidothiomorpholinocarbonylgruppe, eine $C_{1-3}$-Alkylgruppe in 1-, 2- oder 3-Stellung durch eine Arylgruppe oder eine $C_{2-3}$-Alkylgruppe in 2- oder 3-Stellung durch eine Pyrrolidin-2-on-1-yl-, Morpholino-, Thiomorpholino-, 1-Oxido-thiomorpholino- oder 1,1-Dioxidothiomorpholinogruppesubstituiert sein kann, eine Alkenylgruppe mit 1 bis 4 Kohlenstoffatomen, eine Cycloalkyl-, Cycloalkenyl-, Cycloalkylalkyl-, Methoxymethyl- oder Cinnamylgruppe,
R'' ein Wasserstoffatom oder eine Alkylgruppe und
R''' eine geradkettige oder verzweigte Alkylgruppe mit 1 bis 6 Kohlenstoffatomen, eine Cycloalkyl-, Cycloalkylalkyl- oder Arylalkylgruppe bedeuten, wobei eine nach der Abspaltung so gebildete Carboxylgruppe, falls diese an ein Stickstoffatom des Restes A gebunden ist, unter Freisetzung der Amino- oder Iminogruppe decarboxyliert werden kann,
sowie, soweit nichts anderes erwähnt wurde,
unter dem vorstehend erwähnten Begriff "eine Alkylgruppe" eine geradkettige oder verzweigte Alkylgruppe mit 1 bis 3 Kohlenstoffatomen,
unter "eine Cycloalkylgruppe" eine Cycloalkylgruppe mit 5 bis 7 Kohlenstoffatomen,
unter "eine Cycloalkenylgruppe" eine Cycloalkenylgruppe mit 5 bis 7 Kohlenstoffatomen,
unter "eine Alkoxygruppe" eine geradkettige oder verzweigte Alkoxygruppe mit 1 bis 3 Kohlenstoffatomen und unter "eine Arylgruppe" eine durch Fluor-, Chlor- oder Bromatome, durch Alkylgruppen mit 1 bis 5 Kohlenstoffatomen, durch Hydroxy-, Alkoxy-, Phenylalkoxy-, Trifluormethyl-, Mercapto-, Alkylsulfenyl-, Alkylsulfinyl-, Alkylsulfonyl-, Amino-, Alkylamino-, Dialkylamino-, Alkylcarbonylamino-, Phenylalkylcarbonylamino-, Phenylcarbonylamino-, Alkoxycarbonylamino-, Alkylsulfonylamino-, Phenylsulfonylamino-, N-Alkylcarbonyl-alkylamino-, N-Phenylalkylcarbonyl-alkylamino-, N-Phenylcarbonyl-alkylamino-, N-Alkoxycarbonyl-alkylamino-, N-Alkylsulfonyl-alkylamino-, N-Phenylsulfonylalkylamino-, Cyano-, Aminocarbonyl-, Alkylaminocarbonyl-, Dialkylaminocarbonyl-, Aminosulfonyl-, Alkylaminosulfonyl-, Dialkylaminosulfonyl-, Alkylcarbonyl-, Phenylalkylcarbonyl-, Phenylcarbonyl-, Carboxy-, Sulfo-, Alkoxycarbonyl-, Aminocarbonylamino-, N-Aminocarbonylalkylamino- oder Aminoalkylgruppen mono-, di- oder trisubstituierte Phenylgruppe sein kann, wobei die Substituenten gleich oder verschieden sein können und die Aminogruppe bei den vorstehend erwähnten Aminocarbonylamino-, N-Aminocarbonyl-alkylamino- oder Aminoalkylgruppen zusätzlich durch Alkyl- oder Phenylalkylgruppen mono- oder disubstituiert sein kann,
zu verstehen ist,
deren Stereoisomere, deren Gemische und deren Salze.

**2.** Cyclische Iminoderivate der allgemeinen Formel I gemäß Anspruch 1, in der
einer der Reste $R_a$ oder $R_b$ eine A-B-Gruppe und
der andere der Reste $R_a$ oder $R_b$ eine F-E-D-Gruppe, in denen
A eine Aminoalkylgruppe mit 1 bis 4 Kohlenstoffatomen im Alkylteil, eine Amino- oder Amidinogruppe, wobei in den vorstehend erwähnten Amino-, Aminoalkyl- oder Amidinogruppen eine Amino- oder Iminogruppe durch eine Alkylgruppe oder durch eine in vivo abspaltbare Gruppe substituiert sein kann,
B eine Phenylengruppe, die durch ein Fluor-, Chlor- oder Bromatom, durch eine Alkylgruppe, durch eine Trifluormethyl-, Hydroxy-, Alkoxy-, Alkylsulfenyl-, Alkylsulfinyl-, Alkylsulfonyl-, $(R_1)_2$N-, N-Alkylcarbonyl-NR$_1$- oder N-Alkansulfonyl-NR$_1$-Gruppe, wobei $R_1$ ein Wasserstoffatom, eine Alkyl- oder Phenylalkylgruppe darstellt, substituiert sein kann,

eine Pyridinylen-, Pyrimidinylen-, Pyrazinylen- oder Pyridazinylengruppe, die jeweils im Kohlenstoffgerüst durch eine Alkylgruppe substituiert sein können,

eine 1,3-Pyrrolidinylen-, 1,3-Piperidinylen-, 1,4-Piperidinylen-, 1,4-Piperazinylen- oder 1,4-Homopiperazinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf, und

eine Cycloalkylengruppe, in der eine Methylengruppe durch eine $-NR_1$-Gruppe, in der $R_1$ wie vorstehend erwähnt definiert ist, ersetzt sein kann, wobei zusätzlich zwischen dem Stickstoffatom der $-NR_1$-Gruppe und einem Stickstoffatom des Restes A oder des cyclischen Iminorestes mindestens zwei Kohlenstoffatome liegen müssen, und

eine Indanyliden- oder 1,2,3,4-Tetrahydronaphthylidengruppe, wobei jeweils der aromatische Kern mit dem cyclischen Iminorest verknüpft ist, oder

A und B zusammen eine gegebenenfalls in 1-Stellung durch den Rest $R_1$ oder durch eine in vivo abspaltbare Gruppe substituierte Pyrrolidinyl-, Piperidinyl- oder Piperazinogruppe, wobei $R_1$ wie vorstehend erwähnt definiert ist,

D eine Phenylengruppe, die durch ein Fluor-, Chlor- oder Bromatom, durch eine Alkylgruppe, durch eine Trifluormethyl-, Hydroxy-, Alkoxy-, Alkylsulfenyl-, Alkylsulfinyl-, Alkylsulfonyl-, $(R_1)_2N$-, N-Alkylcarbonyl-$NR_1$-, N-Alkansulfonyl-$NR_1$-, Carboxymethoxy- oder Alkoxycarbonylmethoxygruppe substituiert sein kann, wobei $R_1$ wie vorstehend erwähnt definiert ist, substituiert sein kann,

eine Cycloalkylengruppe,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine $-CH=N$-Gruppe durch eine $-CO-NR_2$-Gruppe ersetzt sein kann, wobei $R_2$ ein Wasserstoffatom, eine Alkyl- oder Phenylalkylgruppe oder auch eine Bindung mit dem Rest E darstellt, wenn sich immer zwischen einem Heteroatom des Restes E oder dem Stickstoffatom des cyclischen Iminorestes mit dem der Rest D verknüpft ist und dem Stickstoffatom der $-CONR_2$-Gruppe mindestens zwei Kohlenstoffatome befinden,

eine 1,3-Pyrrolidinylen-, 1,3-Piperidinylen-, 1,4-Piperidinylen-, 1,4-Piperazinylen- oder 1,4-Homopiperazinylengruppe, welche über die Position 1 mit dem Rest E oder mit dem cyclischen Iminorest verknüpft sein können und in denen eine zu dem Stickstoffatom benachbarte Methylengruppe durch eine Carbonylgruppe ersetzt sein kann, wobei jedoch die Verknüpfung nicht über eine Stickstoff-Stickstoff-Bindung erfolgen darf,

eine Cycloalkylengruppe, in der eine Methylengruppe durch eine $-NR_1$-Gruppe ersetzt ist, wobei $R_1$ wie vorstehend erwähnt definiert ist,

E eine gegebenenfalls durch eine Hydroxy-, Alkoxy-, Amino-, Alkylamino-, Dialkylamino-, Alkyl-CO-$NR_1$-, Aryl-CO-$NR_1$-, $C_{1-5}$-Alkyl-$SO_2$-$NR_1$- oder Aryl-$SO_2$-$NR_1$-Gruppe substituierte geradkettige oder verzweigte Alkylengruppe mit 1 bis 5 Kohlenstoffatomen, in denen $R_1$ wie vorstehend erwähnt definiert ist, eine geradkettige oder verzweigte Alkenylengruppe mit 2 bis 5 Kohlenstoffatomen, wobei in einer Alkylengruppe mit 2 bis 4 Kohlenstoffatomen die mit dem Rest D verknüpfte Methylengruppe durch ein Sauerstoff- oder Schwefelatom, durch eine Sulfinyl-, Sulfonyl-, Imino-, Alkylimino-, Phenylalkylimino-, Alkylcarbonylimino-, Carboxymethylimino- oder Alkoxycarbonylmethyliminogruppe ersetzt sein kann, und

F eine in vivo zu einer Carboxylgruppe metabolisierbare Gruppe, eine Carboxylgruppe, eine Sulfo-, Phosphono-, O-Alkylphosphono- oder Tetrazol-5-ylgruppe darstellen, und entweder

(a) X eine Methylen- oder Carbonylgruppe,

$R_d$ und $R_e$ zusammen eine weitere Kohlenstoff-Kohlenstoff-Bindung,

$R_c$ ein Wasserstoffatom, eine Alkylgruppe mit 1 bis 5 Kohlenstoffatomen, eine Phenylgruppe, eine Hydroxygruppe, eine geradkettige oder verzweigte Alkoxygruppe mit 1 bis 5 Kohlenstoffatomen, wobei eine Methoxygruppe durch eine Carboxy-, Alkoxycarbonyl-, Aminocarbonyl-, Alkylaminocarbonyl-, Dialkylaminocarbonyl-, Piperidinocarbonyl-, Morpholinocarbonyl-, Thiomorpholinocarbonyl-, 1-Oxidothiomorpholinocarbonyl- oder 1,1-Dioxidothiomorpholinocarbonylgruppe substituiert sein kann, eine Phenylalkoxy-, Alkylamino-, Dialkylamino-, Phenylalkylamino-, Pyrrolidino- oder Piperidinogruppe, wobei die Methylengruppe in 4-Stellung einer Piperidinogruppe durch ein Sauerstoff- oder Schwefelatom, durch eine Sulfinyl-, Sulfonyl-, Imino-, Alkylimino- oder Alkylcarbonyliminogruppe ersetzt sein kann,

$R_f$ ein Wasserstoffatom, eine geradkettige oder verzweigte Alkylgruppe oder eine Phenylgruppe und

$R_g$ ein Wasserstoffatom oder eine Alkylgruppe oder

(b) X eine Carbonylgruppe,

$R_c$ und $R_e$ zusammen mit dem dazwischen liegenden Kohlenstoffatom eine Carbonylgruppe,

$R_d$ eine Alkylgruppe, eine Phenylalkylgruppe oder eine gegebenenfalls durch eine Carboxy-, Alkoxycarbonyl-, Aminocarbonyl-, Alkylaminocarbonyl-, Dialkylaminocarbonyl-, Piperidinocarbonyl-, Morpholinocarbonyl-, Thiomorpholinocarbonyl-, 1-Oxidothiomorpholinocarbonyl- oder 1,1-Dioxidothiomorpholinocarbonylgruppe substituierte Methylgruppe,
$R_f$ ein Wasserstoffatom, eine Alkyl- oder Phenylgruppe und
$R_g$ ein Wasserstoffatom oder eine Methylgruppe oder
(c) X eine Methylengruppe,
$R_f$ und $R_g$ zusammen mit dem dazwischen liegenden Kohlenstoffatom eine Carbonylgruppe,
$R_c$ ein Wasserstoffatom oder auch, wenn B oder D eine 1,3-Pyrrolidinylen-, 1,3-Piperidinylen-, 1,4-Piperidinylen-, 1,4-Piperazinylen- oder 1,4-Homopiperazinylengruppe, welche über die Position 1 mit dem cyclischen Iminorest verknüpft sind, wobei jedoch die Verbindung nicht über eine Stickstoff-Stickstoff-Bindung erfolgen darf, darstellt, eine Phenylgruppe und
$R_d$ und $R_e$ jeweils ein Wasserstoffatom oder zusammen eine weitere Kohlenstoff-Kohlenstoff-Bindung oder
(d) X eine Methylen- oder Carbonylgruppe und
$R_c$ bis $R_f$ jeweils ein Wasserstoffatom bedeuten,
wobei unter "eine in vivo zu einer Carboxylgruppe metabolisierbare Gruppe" und unter "eine in vivo abspaltbare Gruppe" beispielsweise eine Estergruppe der Formeln

- CO - OR',
- CO - O - (HCR'') - O - CO - R''' und
- CO - O - (HCR'') - O - CO - OR''',

in denen
R' eine geradkettige oder verzweigte Alkylgruppe mit 1 bis 6 Kohlenstoffatomen, wobei die Methylgruppe durch eine Pyridyl-, Aminocarbonyl-, Alkylaminocarbonyl-, Dialkylaminocarbonyl-, Morpholinocarbonyl-, Thiomorpholinocarbonyl-, 1-Oxidothiomorpholinocarbonyl- oder 1,1-Dioxidothiomorpholinocarbonylgruppe, eine $C_{1-3}$-Alkylgruppe in 1-, 2- oder 3-Stellung durch eine Phenylgruppe oder eine $C_{2-3}$-Alkylgruppe in 2- oder 3-Stellung durch eine Morpholino-, Thiomorpholino-, 1-Oxido-thiomorpholino- oder 1,1-Dioxido-thiomorpholinogruppe substituiert sein kann, eine Allyl-, Cycloalkyl- oder Cinnamylgruppe,
R'' ein Wasserstoffatom oder eine Alkylgruppe und
R''' eine geradkettige oder verzweigte Alkylgruppe mit 1 bis 6 Kohlenstoffatomen, eine Cycloalkyl-, Cycloalkylalkyl- oder Phenylalkylgruppe bedeuten, zu verstehen ist, wobei eine nach der Abspaltung so gebildete Carboxylgruppe, falls diese an ein Stickstoffatom des Restes A gebunden ist, unter Freisetzung der Amino- oder Iminogruppe decarboxyliert werden kann,
wobei die bei der Definiton der vorstehenden Reste erwähnten Alkyl- und Alkoxyteile jeweils 1 bis 3 Kohlenstoffatome und die Cycloalkylteile jeweils 5 oder 6 Kohlenstoffatome enthalten können,
deren Stereoisomere, deren Gemische und deren Salze.

**3.** Cyclische Iminoderivate der allgemeinen Formel I gemäß Anspruch 1, in der
einer der Reste $R_a$ oder $R_b$ eine A-B-Gruppe und
der andere der Reste $R_a$ oder $R_b$ eine F-E-D-Gruppe, in denen

A eine Aminoalkylgruppe mit 1 bis 4 Kohlenstoffatomen im Alkylteil, eine Amino- oder Amidinogruppe, wobei in den vorstehend erwähnten Amino-, Aminoalkyl- oder Amidinogruppen eine Amino- oder Iminogruppe durch eine Methylgruppe oder durch eine in vivo abspaltbare Gruppe substituiert sein kann,

B eine Phenylengruppe, die durch eine Methylgruppe, durch ein Fluor-, Chlor- oder Bromatom substituiert sein kann, eine Pyridinylen-, 1,3-Piperidinylen- oder 1,4-Piperidinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf,

eine Cyclohexylengruppe,

eine Indanyliden- oder 1,2,3,4-Tetrahydronaphthylidengruppe, wobei jeweils der aromatische Kern mit dem cyclischen Iminorest verknüpft ist, oder

A und B zusammen eine Piperidinylgruppe,

D eine Phenylengruppe, die durch ein Fluor-, Chlor- oder Bromatom, durch eine Carboxymethoxy- oder Methoxycarbonylmethoxygruppe substituiert sein kann,

eine Cyclohexylen-, Pyridinylen-, Pyrimidinylen-, 1,3-Piperidinylen- oder 1,4-Piperidinylengruppe,

welche über die Position 1 mit dem Rest A oder mit dem cyclischen Iminorest verknüpft sein können, wobei jedoch die Verknüpfung nicht über eine Stickstoff-Stickstoff-Bindung erfolgen darf,

E eine gegebenenfalls durch eine Hydroxy-, Methoxy-, Amino-, Acetylamino- oder Methansulfonylaminogruppe substituierte geradkettige oder verzweigte Alkylengruppe mit 1 bis 4 Kohlenstoffatomen, wobei in einer Alkylengruppe mit 2 oder 3 Kohlenstoffatomen die mit dem Rest D verknüpfte Methylengruppe durch ein Sauerstoff- oder Schwefelatom, durch eine Sulfinyl-, Sulfonyl-, Imino-, Methylimino-, Carboxymethylimino- oder Methoxycarbonylmethyliminogruppe ersetzt sein kann, oder eine Alkenylengruppe mit 2 oder 3 Kohlenstoffatomen und

F eine in vivo zu einer Carboxylgruppe metabolisierbare Gruppe, eine Carboxylgruppe, eine Phosphono-, O-Methylphosphono- oder Tetrazol-5-ylgruppe darstellen, und entweder

(a) X eine Carbonylgruppe,

$R_d$ und $R_e$ zusammen eine weitere Kohlenstoff-Kohlenstoff-Bindung,

$R_c$ ein Wasserstoffatom, eine Phenylgruppe, eine Hydroxygruppe, eine geradkettige oder verzweigte Alkoxygruppe mit 1 bis 3 Kohlenstoffatomen, eine Phenylmethoxy-, Morpholinocarbonylmethoxy-, Methylamino-, Dimethylamino-, Phenylamino-, Pyrrolidino- oder Piperidinogruppe, wobei die Methylengruppe in 4-Stellung einer Piperidinogruppe durch ein Sauerstoff- oder Schwefelatom, durch eine Sulfinyl-, Sulfonyl-, Imino- oder Acetyliminogruppe ersetzt sein kann,

$R_f$ ein Wasserstoffatom, eine Methylgruppe oder eine Phenylgruppe und

$R_g$ ein Wasserstoffatom oder eine Methylgruppe oder

(b) X eine Carbonylgruppe,

$R_c$ und $R_e$ zusammen mit dem dazwischen liegenden Kohlenstoffatom eine Carbonylgruppe,

$R_d$ eine Methyl-, Phenylmethyl- oder Morpholinocarbonylmethylgruppe,

$R_f$ und $R_g$ jeweils ein Wasserstoffatom oder

(c) X eine Methylengruppe,

$R_f$ und $R_g$ zusammen mit dem dazwischen liegenden Kohlenstoffatom eine Carbonylgruppe,

$R_c$, $R_d$ und $R_e$ jeweils ein Wasserstoffatom oder

$R_c$ ein Wasserstoffatom oder auch, wenn B oder D eine 1,3-Piperidinylen- oder 1,4-Piperidinylengruppe, welche über die Position 1 mit dem cyclischen Iminorest verknüpft sind, darstellt, eine Phenylgruppe und

$R_d$ und $R_e$ zusammen eine weitere Kohlenstoff-Kohlenstoff-Bindung oder

(d) X eine Methylen- oder Carbonylgruppe und

$R_c$ bis $R_f$ jeweils ein Wasserstoffatom bedeuten,

wobei unter "eine in vivo zu einer Carboxylgruppe metabolisierbare Gruppe" und unter "eine in vivo abspaltbare Gruppe" beispielsweise eine Estergruppe der Formeln

- CO - OR',
- CO - O - (HCR'') - O - CO - R''' und
- CO - O - (HCR'') - O - CO - OR''',

in denen

R' eine geradkettige oder verzweigte Alkylgruppe mit 1 bis 3 Kohlenstoffatomen, wobei die Methylgruppe durch eine Dimethylaminocarbonyl-, Piperidinocarbonyl- oder Morpholinocarbonylgruppe substituiert sein kann, eine Allyl-, Phenylmethyl-, 2-Morpholinoethyl- oder Cyclohexylgruppe,

R'' ein Wasserstoffatom oder eine Methylgruppe und

R''' eine geradkettige oder verzweigte Alkylgruppe mit 1 bis 4 Kohlenstoffatomen oder eine Cyclohexylgruppe bedeuten, zu verstehen ist, wobei eine nach der Abspaltung so gebildete Carboxylgruppe, falls diese an ein Stickstoffatom des Restes A gebunden ist, unter Freisetzung der Amino- oder Iminogruppe decarboxyliert werden kann, bedeuten,

deren Stereoisomere, deren Gemische und deren Salze.

**4.** Cyclische Iminoderivate der allgemeinen Formel I gemäß Anspruch 1, in der
einer der Reste $R_a$ oder $R_b$ eine A-B-Gruppe und
der andere der Reste $R_a$ oder $R_b$ eine F-E-D-Gruppe, in denen

A eine Aminoalkylgruppe mit 1 bis 4 Kohlenstoffatomen im Alkylteil, eine Amino- oder Amidinogruppe, wobei in den vorstehend erwähnten Amino-, Aminoalkyl- oder Amidinogruppen eine Amino- oder Iminogruppe durch eine Methylgruppe oder durch eine in vivo abspaltbare Gruppe substituiert sein kann,

B eine Phenylengruppe, die durch eine Methylgruppe, durch ein Fluor-, Chlor- oder Bromatom

substituiert sein kann, eine Pyridinylen-, 1,3-Piperidinylen- oder 1,4-Piperidinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf,

eine Cyclohexylengruppe,

eine Indanyliden- oder 1,2,3,4-Tetrahydronaphthylidengruppe, wobei jeweils der aromatische Kern mit dem cyclischen Iminorest verknüpft ist, oder

A und B zusammen eine Piperidinylgruppe,

D eine Phenylengruppe, die durch ein Fluor-, Chlor- oder Bromatom, durch eine Carboxymethoxy- oder Methoxycarbonylmethoxygruppe substituiert sein kann,

eine Cyclohexylen-, Pyridinylen-, Pyrimidinylen-, 1,3-Piperidinylen- oder 1,4-Piperidinylengruppe, welche über die Position 1 mit dem Rest A oder mit dem cyclischen Iminorest verknüpft sein können, wobei jedoch die Verknüpfung nicht über eine Stickstoff-Stickstoff-Bindung erfolgen darf,

E eine gegebenenfalls durch eine Hydroxy-, Methoxy- oder Aminogruppe substituierte geradkettige oder verzweigte Alkylengruppe mit 1 bis 4 Kohlenstoffatomen, wobei in einer Alkylengruppe mit 2 oder 3 Kohlenstoffatomen die mit dem Rest D verknüpfte Methylengruppe durch ein Sauerstoff- oder Schwefelatom, durch eine Sulfinyl-, Sulfonyl-, Imino-, Methylimino-, Carboxymethylimino- oder Methoxycarbonylmethyliminogruppe ersetzt sein kann, oder eine Alkenylengruppe mit 2 oder 3 Kohlenstoffatomen und

F eine in vivo zu einer Carboxylgruppe metabolisierbare Gruppe, eine Carboxylgruppe, eine Phosphono-, O-Methylphosphono- oder Tetrazol-5-ylgruppe darstellen, und entweder

(a) X eine Carbonylgruppe,

$R_d$ und $R_e$ zusammen eine weitere Kohlenstoff-Kohlenstoff-Bindung,

$R_c$ ein Wasserstoffatom, eine Phenylgruppe, eine Hydroxygruppe, eine geradkettige oder verzweigte Alkoxygruppe mit 1 bis 3 Kohlenstoffatomen, eine Phenylmethoxy-, Morpholinocarbonylmethoxy-, Methylamino-, Dimethylamino-, Phenylamino-, Pyrrolidino- oder Piperidinogruppe, wobei die Methylengruppe in 4-Stellung einer Piperidinogruppe durch ein Sauerstoff- oder Schwefelatom, durch eine Sulfinyl-, Sulfonyl-, Imino- oder Acetyliminogruppe ersetzt sein kann,

$R_f$ ein Wasserstoffatom, eine Methylgruppe oder eine Phenylgruppe und

$R_g$ ein Wasserstoffatom oder eine Methylgruppe oder

(b) X eine Carbonylgruppe,

$R_c$ und $R_e$ zusammen mit dem dazwischen liegenden Kohlenstoffatom eine Carbonylgruppe,

$R_d$ eine Methyl-, Phenylmethyl- oder Morpholinocarbonylmethylgruppe,

$R_f$ und $R_g$ jeweils ein Wasserstoffatom oder

(d) X eine Carbonylgruppe und

$R_c$ bis $R_f$ jeweils ein Wasserstoffatom bedeuten,

wobei unter "eine in vivo zu einer Carboxylgruppe metabolisierbare Gruppe" und unter "eine in vivo abspaltbare Gruppe" beispielsweise eine Estergruppe der Formeln

- CO - OR',
- CO - O - (HCR'') - O - CO - R''' und
- CO - O - (HCR'') - O - CO - OR''',

in denen

R' eine geradkettige oder verzweigte Alkylgruppe mit 1 bis 3 Kohlenstoffatomen, wobei die Methylgruppe durch eine Dimethylaminocarbonyl-, Piperidinocarbonyl- oder Morpholinocarbonylgruppe substituiert sein kann, eine Allyl-, Phenylmethyl-, 2-Morpholinoethyl- oder Cyclohexylgruppe,

R'' ein Wasserstoffatom oder eine Methylgruppe und

R''' eine geradkettige oder verzweigte Alkylgruppe mit 1 bis 4 Kohlenstoffatomen oder eine Cyclohexylgruppe bedeuten, zu verstehen ist, wobei eine nach der Abspaltung so gebildete Carboxylgruppe, falls diese an ein Stickstoffatom des Restes A gebunden ist, unter Freisetzung der Amino- oder Iminogruppe decarboxyliert werden kann, bedeuten,

deren Stereoisomere, deren Gemische und deren Salze.

**5.** Cyclische Iminoderivate der allgemeinen Formel I gemäß Anspruch 1, in der einer der Reste $R_a$ oder $R_b$ eine A-B-Gruppe und der andere der Reste $R_a$ oder $R_b$ eine F-E-D-Gruppe, in denen

A eine Aminomethyl- oder Amidinogruppe,

B eine Phenylengruppe,

D eine Phenylengruppe,
E eine Ethylengruppe und
F eine in vivo zu einer Carboxylgruppe metabolisierbare Gruppe oder eine Carboxylgruppe darstellen, und entweder
(a) X eine Carbonylgruppe,
$R_d$ und $R_e$ zusammen eine weitere Kohlenstoff-Kohlenstoff-Bindung,
$R_c$ eine Hydroxy-, Methoxy-, Ethoxy- oder Morpholinogruppe,
$R_f$ und $R_g$ jeweils ein Wasserstoffatom oder
(b) X eine Carbonylgruppe,
$R_c$ und $R_e$ zusammen mit dem dazwischen liegenden Kohlenstoffatom eine Carbonylgruppe,
$R_d$ eine Methylgruppe,
$R_f$ und $R_g$ jeweils ein Wasserstoffatom oder
(d) X eine Carbonylgruppe und
$R_c$ bis $R_f$ jeweils ein Wasserstoffatom bedeuten,
wobei unter "eine in vivo zu einer Carboxylgruppe metabolisierbare Gruppe" eine R'O-CO-Gruppe, in der R' eine geradkettige oder verzweigte Alkylgruppe mit 1 bis 3 Kohlenstoffatomen darstellt, zu verstehen ist, bedeuten,
deren Stereoisomere, deren Gemische und deren Salze.

6. Folgende cyclische Iminoderivate der allgemeinen Formel I gemäß Anspruch 1:
(a) 1-(4-Amidinophenyl)-3-[4-(2-carboxy-ethyl)-phenyl]-4-methoxy-3-pyrrolin-2-on,
(b) 1-(4-Amidinophenyl)-3-[4-(2-carboxy-ethyl)-phenyl]-4-morpholino-3-pyrrolin-2-on,
(c) 1-(4-Amidinophenyl)-3-[4-(2-carboxy-ethyl)-phenyl]-pyrrolidin-2-on,
(d) 1-(4-Aminomethyl-phenyl)-3-[4-(2-carboxy-ethyl)-phenyl]-4-methoxy-3-pyrrolin-2-on und
(e) 1-(4-Amidinophenyl)-3-[4-(2-carboxy-ethyl)-phenyl]-3-methyl-pyrrolidin-2,4-dion,
deren Stereoisomere, deren Gemische und deren Salze.

7. Physiologisch verträgliche Salze der Verbindungen nach mindestens einem der Ansprüche 1 bis 6 mit anorganischen oder organischen Säuren oder Basen.

8. Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

9. Verwendung einer Verbindung nach mindestens einem der Ansprüche 1 bis 7 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

10. Verfahren zur Herstellung eines Arzneimittels gemäß Anspruch 8, dadurch gekennzeichnet, daß auf nichtchemischem Wege eine Verbindung nach mindestens einem der Ansprüche 1 bis 7 in einen oder mehrere inerte Trägerstoffe und/oder Verdünnungsmittel eingearbeitet wird.

11. Verfahren zur Herstellung der cyclischen Iminoderivate gemäß den Ansprüchen 1 bis 7, dadurch gekennzeichnet, daß
a) zur Herstellung von Verbindungen der allgemeinen Formel I, in der F eine Carboxylgruppe darstellt, eine Verbindung der allgemeinen Formel

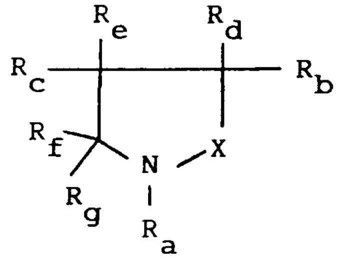

,(II)

in der
X und $R_a$ bis $R_g$ mit der Maßgabe wie in den Ansprüchen 1 bis 6 definiert sind, daß einer der Reste $R_a$ oder $R_b$ eine A-B-Gruppe und der andere der Reste $R_a$ oder $R_b$ eine F'-E-D-Gruppe bedeutet, wobei B, E und D wie in den Ansprüchen 1 bis 6 definiert sind und F' eine mittels Hydrolyse, Behandeln mit Säuren, Thermolyse oder Hydrogenolyse in eine Carboxylgruppe überführbare Gruppe bedeutet, in eine Verbindung der allgemeinen Formel I, in der F eine Carboxylgruppe darstellt, umgewandelt wird oder

b) zur Herstellung von Verbindungen der allgemeinen Formel I, in der A eine gegebenenfalls an der Aminogruppe durch eine oder zwei Alkylgruppen substituierte $H_2N$-C(=NH)-Gruppe darstellt, eine gegebenenfalls im Reaktionsgemisch gebildeten Verbindung der allgemeinen Formel

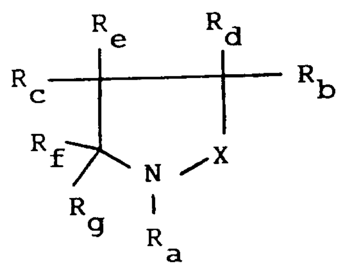

,(III)

in der
X und $R_a$ bis $R_g$ mit der Maßgabe wie in den Ansprüchen 1 bis 6 definiert sind, daß einer der Reste $R_a$ oder $R_b$ eine NC-B-Gruppe und der andere der Reste $R_a$ oder $R_b$ eine F-E-D-Gruppe bedeutet, wobei B, E und D wie in den Ansprüchen 1 bis 6 definiert sind, mit einem Amin der allgemeinen Formel

$(R_1')_2NH$ ,(IV)

in der
die Reste $R_1'$, die gleich oder verschieden sein können, Wasserstoffatome oder Alkylgruppen mit jeweils 1 bis 4 Kohlenstoffatomen darstellen, oder mit deren Säureadditionssalzen umgesetzt wird oder

c) zur Herstellung von Verbindungen der allgemeinen Formel I, in der mindestens einer der Reste $R_a$ bis $R_g$ eine Sulfinyloder Sulfonylgruppe enthält, eine Verbindung der allgemeinen Formel

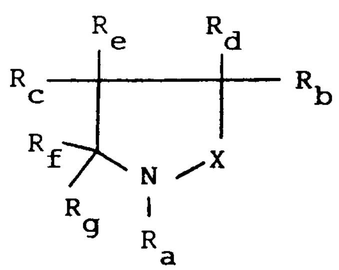

,(V)

in der
X und $R_a$ bis $R_g$ mit der Maßgabe wie in den Ansprüchen 1 bis 6 definiert sind, daß mindestens einer der Reste $R_a$ bis $R_g$ eine Sulfenyl- oder Sulfinylgruppe enthält, oxidiert wird oder

d) zur Herstellung von Verbindungen der allgemeinen Formel I, in der A eine Aminogruppe, eine Aminoalkylgruppe mit 1 bis 4 Kohlenstoffatomen im Alkylteil, eine Amidino- oder Guanidinogruppe darstellt, wobei in den vorstehend erwähnten Gruppen die Amino- oder Iminogruppe durch eine Alkylgruppe mit 1 bis 4 Kohlenstoffatomen oder durch eine Phenylalkylgruppe substituiert sein kann, die alle zusätzlich am Stickstoffatom durch eine in vivo abspaltbare Gruppe substituiert sind, eine Verbindung der allgemeinen Formel

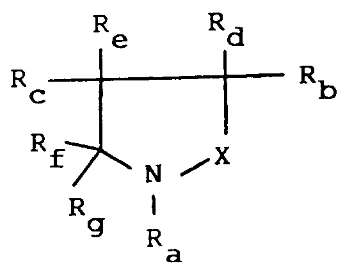

,(VI)

in der
X und $R_a$ bis $R_g$ mit der Maßgabe wie in den Ansprüchen 1 bis 6 definiert sind, daß
einer der Reste $R_a$ oder $R_b$ eine A'-B-Gruppe und
der andere der Reste $R_a$ oder $R_b$ eine F-E-D-Gruppe bedeutet, wobei B, D, E und F wie in den Ansprüchen 1 bis 6 definiert sind und
A' eine Aminogruppe, eine Aminoalkylgruppe mit 1 bis 4 Kohlenstoffatomen im Alkylteil, eine Amidino- oder Guanidinogruppe darstellt, wobei in den vorstehend erwähnten Gruppen die Amino- oder Iminogruppe durch eine Alkylgruppe mit 1 bis 4 Kohlenstoffatomen oder durch eine Phenylalkylgruppe substituiert sein kann, mit einer Verbindung der allgemeinen Formel

$Z_1$ - $R_3$ ,(VII)

in der
$R_3$ eine Gruppe der Formeln

- CO - OR',
- CO - O - (HCR") - O - CO - R"' und
- CO - O - (HCR") - O - CO - OR"' darstellt, in denen

R', R" und R"' wie in den Ansprüchen 1 bis 6 definiert sind, und
$Z_1$ eine nukleophile Austrittsgruppe wie ein Halogenatom oder eine gegebenenfalls substituierte Phenoxygruppe darstellt, umgesetzt wird oder
e) zur Herstellung von Verbindungen der allgemeinen Formel I, in der A eine Aminoalkylgruppe darstellt, eine Verbindung der allgemeinen Formel

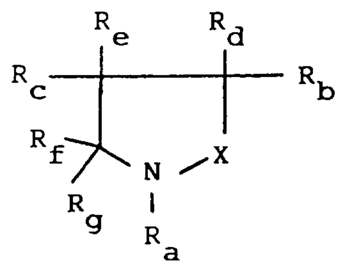

,(VIII)

in der
X und $R_a$ bis $R_g$ mit der Maßgabe wie in den Ansprüchen 1 bis 6 definiert sind, daß
einer der Reste $R_a$ oder $R_b$ eine A"-B-Gruppe und
der andere der Reste $R_a$ oder $R_b$ eine F-E-D-Gruppe bedeutet, wobei B, D, E und F wie in den Ansprüchen 1 bis 6 definiert sind und
A" eine Cyano- oder Cyanoalkylgruppe darstellt, reduziert wird oder
f) zur Herstellung von Verbindungen der allgemeinen Formel I, in der A eine Amidinogruppe und B eine Cycloalkyleniminogruppe mit 4 bis 6 Kohlenstoffatomen darstellen, wobei die Amidinogruppe mit dem Stickstoffatom der Cycloalkyleniminogruppe verknüpft ist, eine Verbindung der allgemeinen Formel in der

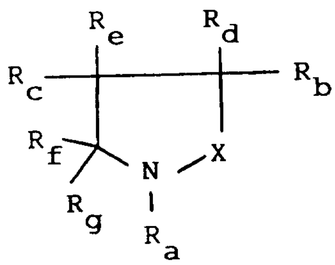

,(IX)

X und $R_a$ bis $R_g$ mit der Maßgabe wie in den Ansprüchen 1 bis 6 definiert sind, daß einer der Reste $R_a$ oder $R_b$ eine H-B"-Gruppe und der andere der Reste $R_a$ oder $R_b$ eine F-E-D-Gruppe bedeutet, wobei D, E und F wie in den Ansprüchen 1 bis 5 definiert sind und B" eine Cycloalkyleniminogruppe mit 4 bis 6 Kohlenstoffatomen darstellt, mit einem S-Alkyl-isothioharnstoff umgesetzt wird oder

g) zur Herstellung der Verbindungen der allgemeinen Formel I, in der $R_e$ und $R_d$ jeweils ein Wasserstoffatom darstellen, eine Verbindung der allgemeinen Formel

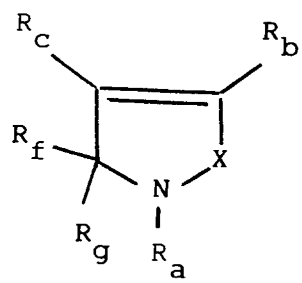

,(X)

in der
X, $R_a$ bis $R_c$, $R_f$ und $R_g$ wie in den Ansprüchen 1 bis 6 definiert sind, hydriert wird oder

h) zur Herstellung von Verbindungen der allgemeinen Formel I, in der F eine R'O-CO-, R'''-CO-O-(HCR")-O-CO- oder R'''O-CO-O-(HCR")-O-CO-Gruppe darstellt, eine Verbindung der allgemeinen Formel

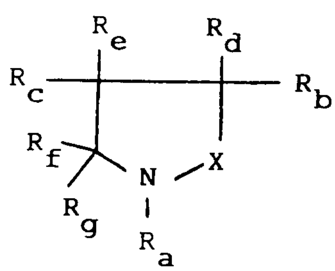

,(XI)

in der
X und $R_a$ bis $R_g$ mit der Maßgabe wie in den Ansprtichen 1 bis 5 definiert sind, daß F eine Carboxygruppe darstellt, oder deren reaktionsfähige Derivate mit einer Verbindung der allgemeinen Formel

$Z_2$ - $R_4$ ,(XII)

in der
$R_4$ eine R'-, R'''-CO-O-(HCR")- oder R'''O-CO-O-(HCR")-Gruppe, wobei R', R" und R''' wie in den Ansprüchen 1 bis 6 definiert sind, und

$Z_2$ eine Hydroxygruppe oder eine nukleophile Austrittsgruppe darstellt, verestert wird und erforderlichenfalls ein während den Umsetzungen zum Schutze von reaktiven Gruppen verwendeter Schutzrest abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 93 10 6726
Seite 1

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| P,Y | EP-A-0 483 667 (DR. KARL THOMAE GMBH) 6. Mai 1992 * Seite 22, Zeile 39 - Seite 22, Zeile 51; Ansprüche 1-12 * --- | 1-11 | C07D207/26<br>C07D207/38<br>A61K31/40 |
| P,Y | EP-A-0 503 548 (DR. KARL THOMAE GMBH) 16. September 1992 * Seite 24, Zeile 5 - Seite 24, Zeile 16; Ansprüche 1-12 * --- | 1-11 | |
| P,Y | EP-A-0 525 629 (DR. KARL THOMAE GMBH) 3. Februar 1993 * Seite 23, Zeile 41 - Seite 23, Zeile 53; Ansprüche 1-11 * --- | 1-11 | |
| P,Y | EP-A-0 528 369 (DR. KARL THOMAE GMBH) 24. Februar 1993 * Seite 20, Zeile 13 - Seite 20, Zeile 29; Ansprüche 1-12 * --- | 1-11 | |
| | -/-- | | |

RECHERCHIERTE SACHGEBIETE (Int. Cl. 5)

C07D
A61K

## UNVOLLSTÄNDIGE RECHERCHE

**Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Siehe Ergänzungsblatt C

| Recherchenort | Abschlußdatum der Recherche | Prüfer |
|---|---|---|
| MUENCHEN | 13 AUGUST 1993 | HERZ C.P. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER TEILRECHERCHENBERICHT

Nummer der Anmeldung

EP 93 10 6726
Seite 2

| EINSCHLÄGIGE DOKUMENTE | | | KLASSIFIKATION DER ANMELDUNG (Int. Cl. 5) |
|---|---|---|---|
| Kategorie | Kennzeichnung des Dokuments mit Angabe, soweit erforderlich der maßgeblichen Teile | Betrifft Anspruch | |
| Y | EP-A-0 409 163 (MERREL DOW PHARMACEUTICALS INC.) 23. Januar 1991 * Ansprüche 1-18 * | 1-11 | |
| Y | EP-A-0 350 437 (SCHERING AG) 10. Januar 1990 * Seite 4, Zeile 31 - Seite 4, Zeile 34; Ansprüche 1-8 * | 1-11 | |
| Y | US-A-4 491 591 (BURROUGHS WELLCOME CO.) 1. Januar 1985 * Spalte 1, Zeile 46 - Spalte 2, Zeile 24; Ansprüche 1,2 * | 1-11 | RECHERCHIERTE SACHGEBIETE (Int. Cl. 5) |
| A | EP-A-0 196 184 (THE WELLCOME FOUNDATION LTD.) 1. Oktober 1986 * gesamtes Dokument * | 1-11 | |
| A | PATENT ABSTRACTS OF JAPAN vol. 014, no. 566 17. Dezember 1990 & JP-A-22 47 166 ( TAISHO PHARMACEUT. CO. LTD. ) 2. Oktober 1990 * Zusammenfassung * | 1-11 | |

EP 93 10 6726

-C-

UNVOLLSTÄNDIGE RECHERCHE

Vollständig recherchierte Patentansprüche: 5,6

Unvollständig recherchierte Patentansprüche 1-4,7-11

Die Definition der folgenden Substituenten sowie die unübersichtliche Verzweigung der einzelnen Variablen in weitere Untereinheiten ist zu allgemein und/oder umfasst einen viel zu breiten Bereich chemisch völlig verschiedener Gruppen, welche im Beschreibungsteil der Anmeldung nicht oder nur zum Teil durch Beispiele veranschaulicht werden:

$R_a$, $R_b$, $R_c$, $R_d$, $R_e$, $R_f$, $R_g$

Die aus diesen Definitionen ableitbare grosse Zahl theoretisch vorstellbarer Verbindungen, welche sich aus den beanspruchten Kombinationsmöglichkeiten ergibt, schliesst eine sinnvolle, vollständige und ökonomische Recherche aus.
Auch im Hinblick auf das in der Beschreibung offenbarte, der Anmeldung zugrundeliegende erfinderische Konzept wurde die Recherche auf den Gegenstand der Ansprüche 5 und 6 und die konkret offenbarten Ausführungsbeispiele beschränkt.

(s.a. Artikel 83,84 EPÜ, Regel 45 l.R., Richtlinien für die Prüfung im EPA, Teil B, III-2.1, 3.6,3.7)